# FASCICULE DE BREVET EUROPEEN

(11) **EP 3 177 667 B1**
(45) Date de publication et mention de la délivrance du brevet: **06.06.2018**
(21) Numéro de dépôt: 15742324.5
(22) Date de dépôt: 31.07.2015
(51) Int. Cl.: C08G 69/28, C08G 69/16, C08G 69/42, C07D 333/32, C07C 323/52, C07C 323/58, C09D 177/02, C09D 177/06

(54) **POLYMERE DOTE DE PROPRIETES D'ADHERENCE**
POLYMER MIT HAFTEIGENSCHAFTEN
POLYMER HAVING ADHERENCE PROPERTIES

(30) Priorité: 08.08.2014 FR 1457711
(43) Date de publication de la demande: 14.06.2017
(73) Titulaire: Xanchem, 91400 Orsay (FR); Ecole Polytechnique, 91128 Palaiseau Cedex (FR)
(72) Inventeur: KALAÏ, Chakib, F-75019 Paris (FR); ZARD, Samir, F-91190 Gif-sur-Yvette (FR); SIRE, Béatrice, F-91120 Palaiseau (FR)
(74) Mandataire: Nony
(86) Numéro de dépôt international: PCT/EP2015/067734
(87) Numéro de publication internationale: WO 2016/020297

(56) Documents cités:
- WO-A1-2006/036646
- GB-A- 568 565
- US-A- 2 377 753

## Description

La présente invention concerne le domaine des polymères utiles pour former des revêtements en surface de supports.

Une grande diversité de supports est aujourd'hui traitée en surface avec un revêtement de nature polymérique. Ces revêtements sont le plus souvent considérés à des fins esthétiques mais aussi de protection, en particulier à l'égard de la corrosion. Ainsi, les substrats de nature métallique, dédiés à être en contact fréquent avec des produits corrosifs tels que les hydrocarbures, les bases et acides minéraux, sont le plus souvent traités en surface avec un revêtement polymérique, principalement de type polyamide. Ces revêtements polymériques sont également mis à profit pour protéger des substrats fragiles, à l'image par exemple du verre, contre les chocs.

Toutefois, les revêtements de surface actuellement disponibles ne donnent pas totale satisfaction, notamment en terme d'adhérence.

Au cours du temps, il est en effet fréquent de constater une perte d'adhérence, localisée ou totale, de ce revêtement avec pour conséquence une fragmentation de celui-ci. Pour des raisons évidentes, cette altération de l'intégrité du revêtement est inesthétique mais peut être également préjudiciable sur le plan de la protection à l'égard du support ainsi mis à découvert et donc devenant vulnérable à la corrosion ou aux chocs mécaniques.

Il demeure donc un besoin de revêtements polymériques dotés de propriétés adhérentes accrues.

Il demeure également un besoin d'une technologie simple et à coût non excessif permettant de conférer à des polymères déjà considérés pour former ces revêtements une adhérence améliorée à l'égard d'une grande diversité de supports.

La présente invention a précisément pour objectif de répondre à ces attentes.

### DEFINITION DE L'INVENTION

Plus particulièrement, les inventeurs ont mis au point des polymères dont la chaîne polymérique a pour originalité et intérêt de posséder, en positions non terminales, des fonctions thiol libres. Les polymères selon l'invention ont ainsi pour intérêt de tirer profit de la grande réactivité des fonctions thiol, et notamment de leur aptitude à se complexer avec certains atomes métalliques.

Ainsi, l'invention décrit un polymère doté de propriétés d'adhérence, notamment à l'égard d'un support organique ou inorganique, caractérisé en ce qu'il comprend au moins un, de préférence plusieurs, motifs structuraux, consécutifs ou non, de formule (I) : 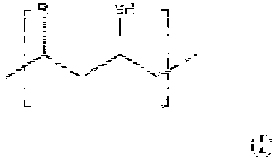 dans laquelle R représente :
- un atome d'hydrogène,
- un radical hydrocarboné en C₁ à C₆, linéaire ou ramifié, saturé ou insaturé et, le cas échéant, substitué par un ou plusieurs atomes d'halogène,
- un radical aryle en C₅ à C₆, ou
- un radical acyle, acyloxy, alcoxycarbonyle ou cyano.

Plus particulièrement, selon un premier aspect, l'invention concerne un polymère doté de propriétés d'adhérence, notamment à l'égard d'un support organique ou inorganique, caractérisé en ce qu'il comprend au moins un, de préférence plusieurs, motifs structuraux, consécutifs ou non, de formule (I) : 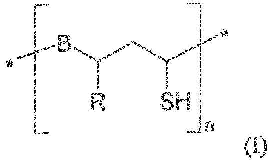 dans laquelle :
- B représente un motif carbonyle ou -R'- avec R' représentant un atome d'hydrogène, un radical alkyle en C₁ à C₆, un radical acyle, carbamate ou aryle en C₅ à C₆ ; et
- R représente :
   - un atome d'hydrogène,
   - un radical hydrocarboné en C₁ à C₆, linéaire ou ramifié, saturé ou insaturé et, le cas échéant, substitué par un ou plusieurs atomes d'halogène,
   - un radical aryle en C₅ à C₆, ou
   - un radical acyle, acyloxy, alcoxycarbonyle ou cyano.

Avantageusement, le polymère selon l'invention possède une quantité efficace en motifs de formule (I) pour lui conférer une propriété tackifante notamment à l'égard d'un support.

En ce qui concerne les supports, ou substrats, ils peuvent être de natures très diverses et être notamment inorganiques, en particulier métalliques ou de type verre ou encore organiques, notamment hydrocarbonés ou siliconés, en particulier avec insaturations ou des motifs sulfhydryles.

En particulier, le polymère selon l'invention répond en partie à la formule (II) ou (III) : 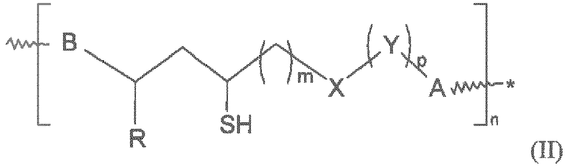 avec n, m, p, R, B, A, Y et X étant tels que définis ci-après,
ou 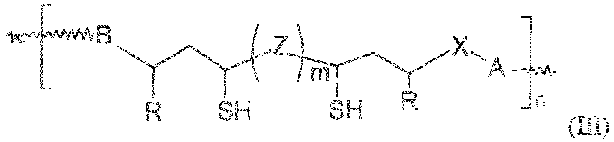 avec n, m, R, B, A, X et Z étant tels que définis ci-après.

Plus avantageusement, un polymère selon l'invention répond en partie à la formule (IIa) ou (IIIa) : 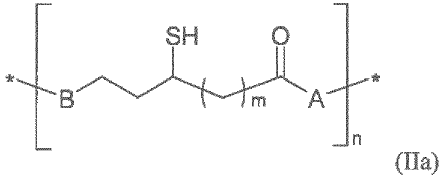 ou 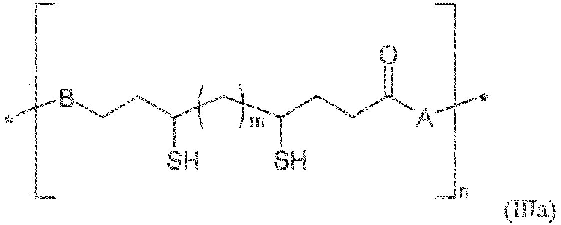 avec n, m, B et A étant tels que définis ci-après.

En particulier, un polymère selon l'invention peut dériver de la condensation, notamment par copolymérisation, d'au moins une entité de formule (II), (IIa), (III) ou (IIIa) avec au moins une chaine polymérique annexe, distincte de ladite entité.

Plus précisément, cette chaîne polymérique distincte est choisie parmi les polyamides, les polyéthers, les chaînes siliconées, les polyamines et les polysulfures.

Selon un autre de ses aspects, l'invention concerne une composition pour revêtement comprenant au moins un polymère selon l'invention.

Selon encore un autre de ses aspects, l'invention concerne un procédé pour former un revêtement en surface d'un support comprenant au moins la mise en contact de la surface à traiter dudit support avec un polymère selon l'invention et l'exposition dudit polymère au contact dudit support à des conditions propices à sa transformation en un revêtement.

Selon un autre de ses aspects, l'invention concerne un support traité en surface avec un revêtement dérivant en tout ou partie d'un polymère selon l'invention.

L'invention vise en outre un procédé utile pour moduler les propriétés en termes d'adhérence d'un polymère comprenant la mise en présence dudit polymère avec au moins un précurseur d'une entité de formule (II), (IIa), (III) ou (IIIa) selon l'invention dans laquelle l'indice « n » a avantageusement une valeur différente de 1 et de préférence possède une valeur représentative d'un oligomère ou polymère, dans des conditions propices à la condensation, et notamment la copolymérisation, dudit précurseur avec ledit polymère à traiter.

### DESCRIPTION DETAILLEE

Comme énoncé ci-dessus, le niveau d'adhérence des polymères selon l'invention repose au moins en partie sur la présence dans sa chaîne polymérique d'un nombre contrôlé de fonctions SH qui y sont intégrées sous la forme de motifs structuraux de formule (I) : 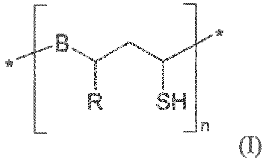 avec B et R étant tels que définis ci-dessus et R représentant de préférence un atome d'hydrogène.

Les différents motifs de formule (I) présents dans un polymère selon l'invention peuvent ou non avoir des groupements R identiques ou différents. De préférence, ils sont identiques.

Avantageusement, les motifs de formule (I) ne sont pas présents au sein du polymère selon l'invention de manière contigüe.

Le ou chaque motif de formule (I) peut y être présent sous la forme d'une entité structurelle, de nature oligomérique, polymérique ou non, avantageusement condensable notamment par copolymérisation.

Selon une première variante de réalisation, un motif de formule (I) est présent dans le polymère selon l'invention sous la forme d'une entité structurelle de formule (II) : 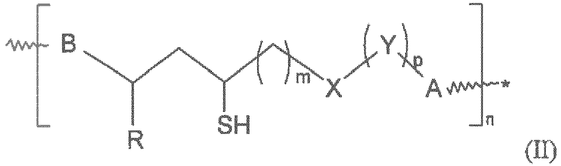 dans laquelle :
- m est un entier variant de 1 à 14 et de préférence égal à 1 ou 8,
- p est égale à zéro ou 1,
- n est un entier représentatif d'un enchaînement oligomérique ou polymériques, en particulier de PM pouvant varier de 2 à 2000,
- R est tel que défini précédemment,
- X représente un motif carbonyle ou NR'- avec R' représentant un atome d'hydrogène ou un radical alkyle en C₁ à C₆,
- Y représente un motif 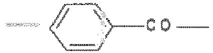 dont la fonction carbonyle est liée à A,
- A représente :
   - un radical de formule -NR₁(R₂)CO- avec :
      * R₂ figurant une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et
      * R₁ représentant un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle, ou
   - un radical de formule -NH(R")NH-, -CO(R")CO-, -O(R")O- ou -S(R")S-, avec R" représentant une chaine hydrocarbonée, linéaire ou ramifié, saturée ou insaturée, polymérique ou non, le cas échéant interrompue par un ou plusieurs hétéroatomes, de préférence des atomes d'oxygène, d'azote ou de soufre, un radical aryle ou aralkyle, et
- B représente un motif carbonyle ou -NR'- avec R' représentant un atome d'hydrogène ou un radical alkyle en C₁ à C₆ avec B étant apte à former un groupement de liaison avec la fonction terminale d'un motif A.

Selon une variante préférée, le polymère selon l'invention comprend au moins une entité structurelle de formule (II) répondant à la formule (IIa) : 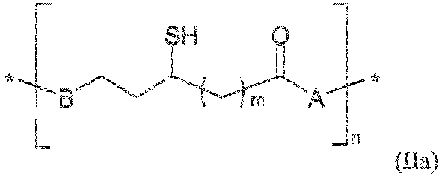 dans laquelle :
- n, m, et B sont tels que définis ci-dessus, et
- A représente :
   - un radical de formule -NH(R₂)CO- avec R₂ figurant une chaîne hydrocarbonée de C₂ à C₁₀, linéaire ou ramifiée, le cas échéant, interrompue par un ou plusieurs hétéroatomes comme des atomes d'oxygène, de soufre ou d'azote,
   - un radical de formule -NH(R")NH- avec R" représentant une chaine hydrocarbonée de C₂ à C₁₀, linéaire ou ramifié, saturée ou insaturée, polymérique ou non, le cas échéant interrompue par un ou plusieurs hétéroatomes, de préférence des atomes d'oxygène, d'azote ou de soufre.

Selon une seconde variante de réalisation, au moins un motif de formule (I) est présent dans le polymère selon l'invention sous la forme d'une entité structurelle de formule (III) : 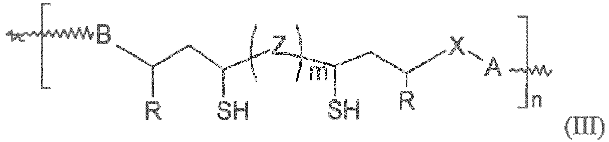 dans laquelle :
- n, m, B, X et R sont tels que définis ci-dessus,
- A représente :
   - un radical de formule -NR₁(R')NR₂- avec R' figurant une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et R₁ et R₂ représentant, indépendamment l'un de l'autre, un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle ;
   - un radical di-yle hétérocyclique pouvant être choisi parmi les radicaux pipérazinyle, 1,3,4,5-tétrahydroimidazolyle, 1,4-diazépanyle ou 1,5-diazocanyle,
   - un radical de formule -CO(R")CO-, -O(R")O- ou -S(R")S- avec R" représentant une chaine hydrocarbonée, linéaire ou ramifiée, saturée ou insaturée, polymérique ou non, le cas échéant interrompue par un ou plusieurs hétéroatomes, de préférence des atomes d'oxygène, d'azote ou de soufre, un radical aryle ou aralkyle, et
- Z représente :
   - un motif hydrocarboné en C₁ à C₂₀, saturé ou insaturé, le cas échéant interrompu par
      - un ou plusieurs hétéroatomes tels que des atomes d'oxygène, de soufre ou d'azote et de préférence un ou plusieurs atomes d'oxygène,
      - un ou plusieurs groupements divalents choisi parmi -NR'- avec R' étant un hydrogène ou un radical alkyle en C₁ à C₆, le cas échéant, substitué par une fonction -SH, et/ou
      - un radical di-yle aryle en particulier phényle, ou
   - une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier du type polyalkylène glycol et, notamment, une chaîne du type polyéthylène glycol ou polysiloxane.

Selon une variante préférée, l'entité de formule (III) est de formule (IIIa) : 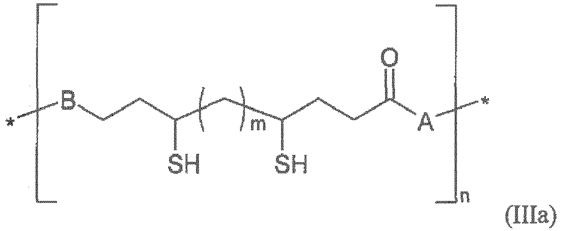 dans laquelle :
- n, m et B sont tels que définis ci-dessus, et
- A représente :
   - un radical de formule -NR₁(R')NR₂- avec R' figurant une chaîne hydrocarbonée de C₂ à C₁₀, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et R₁ et R₂ représentant indépendamment l'un de l'autre un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle, ou
   - un radical di-yle hétérocyclique pouvant être choisi parmi les radicaux pipérazinyle, 1,3,4,5-tétrahydroimidazolyle, 1,4-diazépanyle et 1,5-diazocanyle.

Selon un mode de réalisation particulier, R représente un atome d'hydrogène en formules (II) et (III).

Selon un mode de réalisation particulier, A représente en formule (II) ou (IIa) un radical de formule -NH(R')NH- ou de formule -NH(R₂)CO- avec R' et R₂ figurant une chaîne hydrocarbonée au moins en C₂, polymérique ou non, linéaire ou ramifiée, le cas échéant, interrompue par un ou plusieurs atomes d'oxygène et B représente NH lorsque A représente -NH(R₂)CO- et CO lorsque A représente NH(R')NH-.

En particulier, lorsque A représente un radical -NH(R₂)CO-, R₂ y figure une chaîne hydrocarbonée de C₂ à C₁₀, linéaire ou ramifiée, le cas échéant, interrompue par un ou plusieurs hétéroatomes comme des atomes d'oxygène, de soufre ou d'azote.

Selon un mode de réalisation particulier, A représente en formule (III) ou (IIIa) un radical di-yle hétérocyclique et plus particulièrement un radical di-yle pipérazinyle.

A titre illustratif et non limitatif des structures polymériques ou composés spécifiques conformes à l'invention car possédant au moins un motif de formule (I), voire conformes aux sous-formules (II), (IIa), (III) ou (IIIa) d'entité peuvent notamment être citées les structures qui suivent : 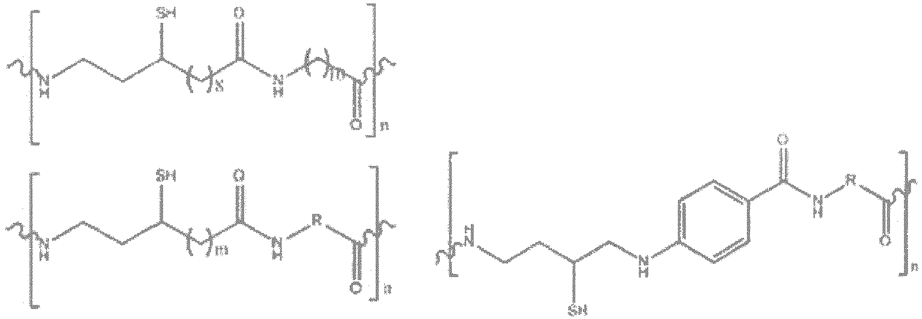 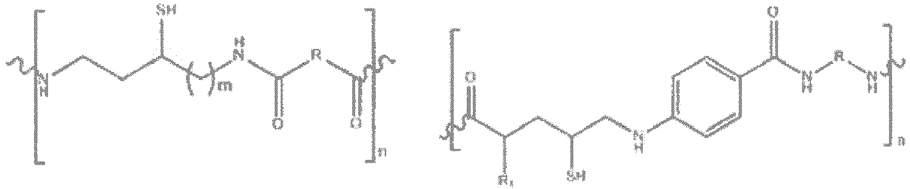 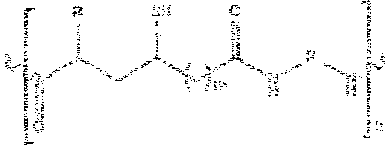 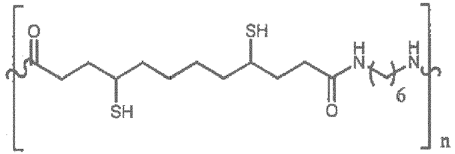 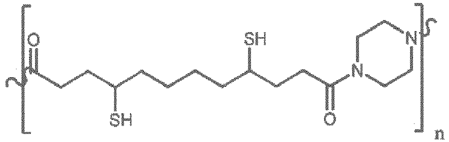 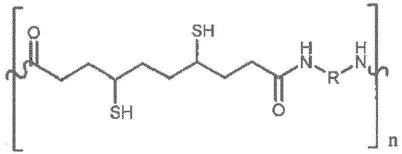 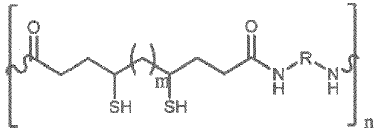 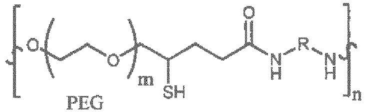 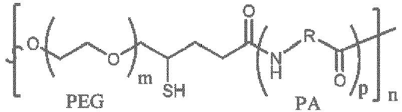 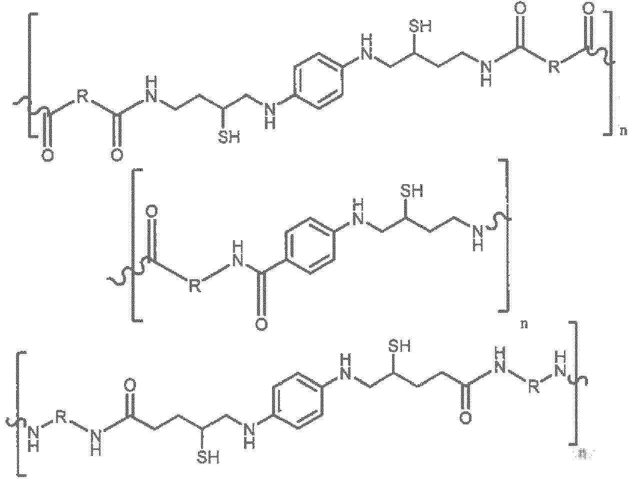 avec :
- R₁ étant conforme à la définition de R en formules (II) et (III),
- R étant conforme à la définition de R₂ dans -NR₁(R₂)CO- ou de R" dans -NH(R")NH-, proposés pour A en formule (II), et
- m et n étant tels que définis en formules (II) et (III).

Plus particulièrement, un polymère selon l'invention peut comprendre au moins une entité structurelle choisie parmi les formules : 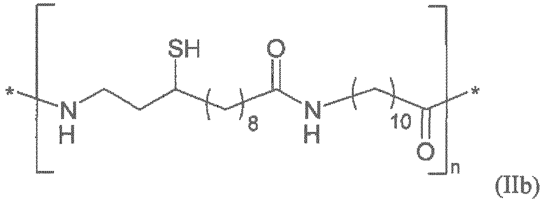 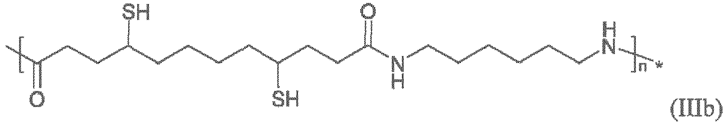 et 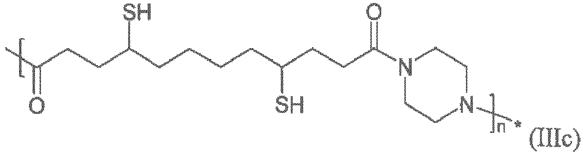 dans laquelle n est tel que défini ci-dessus.

Selon un mode de réalisation, un polymère selon l'invention peut comprendre au moins une entité structurelle de formule (IIb) : 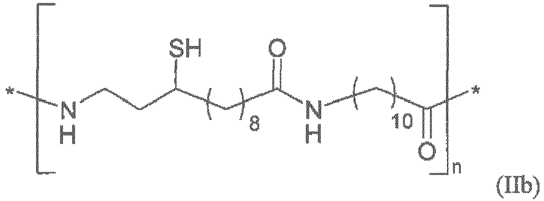 dans laquelle n est tel que défini ci-dessus.

Selon un mode de réalisation, l'entité (III) selon l'invention est de formule (IIIb) : 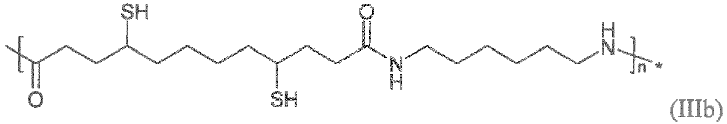 dans laquelle n est tel que défini ci-dessus.

Selon un mode de réalisation, l'entité (III) selon l'invention est de formule (IIIc) : 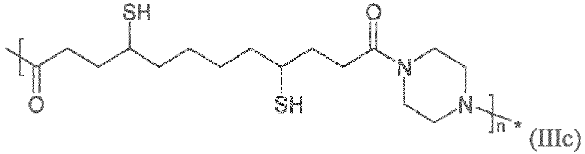 dans laquelle n est tel que défini ci-dessus.

La synthèse des polymères selon l'invention, et en particulier des entités structurelles de formules (II), (IIa), (III) ou (IIIa), relève des compétences de l'homme de l'art.

Un polymère selon l'invention peut ainsi dériver de la copolymérisation de molécules d'un précurseur de l'une des entités de formule (II), (IIa), (III) ou (IIIa) avec au moins un monomère distinct.

Avantageusement la forme précurseur correspond à la forme monomérique de l'une des entités de formule (II), (IIa), (III) ou (IIIa) c'est-à-dire dans laquelle l'indice « n » est égal à 1.

Au sens de la présente invention, un monomère est précurseur d'une entité de formule (II), (IIa), (III) ou (IIIa) dans la mesure où sa condensation, avantageusement sa copolymérisation, avec un monomère distinct conduit, directement ou non, à un oligomère et de préférence à un polymère formé pour l'essentiel d'une entité de formule (II), (IIa), (III) ou (IIIa) dans laquelle l'indice « n » est un entier supérieur à 1 et de préférence possède une valeur représentative d'un oligomère ou polymère.

Par exemple, des polymères reproduisant la structure d'une entité oligomérique ou polymérique de formule (II) ou (IIa) peuvent être obtenus par copolymérisation d'au moins un monomère de formule (IV) : 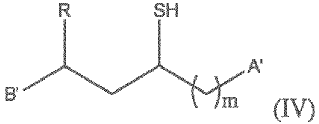 dans laquelle :
- R et m sont tels que définis ci-dessus en formule (II),
- B' et A' représentent respectivement un radical choisi parmi une fonction carboxylique et amine,
avec au moins un monomère choisi parmi H₂N-R-CO₂H, HCO₂R-CO₂H, et H₂N-R-NH₂, dans lesquels R représente une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote.

En ce qui concerne les monomères de formule (IV), ils peuvent être au préalable préparés selon la technologie décrite dans S. Zard et al. (Organic Letters ; 2008, Vol. 10, p. 3 279).

Pour ce qui est des polymères reproduisant la structure d'une entité oligomérique ou polymérique de formule (III) ou (IIIa), ils peuvent notamment être obtenus par copolymérisation d'au moins un monomère de formule (V) : 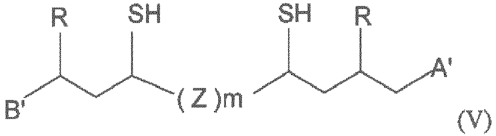 dans laquelle :
- R, Z et m sont tels que définis ci-dessus en formule (III),
- B' et A' sont tels que définis en formule (IV),
avec au moins un monomère choisi parmi H₂N-R-CO₂H, et H₂N-R-NH₂, dans lesquels R représente une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote.

La présente invention a également pour objet un composé de formule (V) : 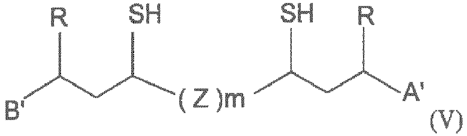 dans laquelle :
- R représente :
   - un atome d'hydrogène,
   - un radical hydrocarboné en C₁ à C₆, linéaire ou ramifié, saturé ou insaturé et, le cas échéant, substitué par un ou plusieurs atomes d'halogène,
   - un radical aryle en C₅ à C₆, ou
   - un radical acyle, acyloxy, alcoxycarbonyle ou cyano,
- Z représente :
   - un motif hydrocarboné en C₁ à C₂₀, saturé ou insaturé, le cas échéant interrompu par :
      - un ou plusieurs hétéroatomes comme des atomes d'oxygène, de soufre ou d'azote et de préférence un ou plusieurs atomes d'oxygène,
      - un ou plusieurs groupements divalents choisi parmi -NR'- avec R' pouvant être un hydrogène ou un radical alkyle en C₁ à C₆, le cas échéant, substitué par une fonction -SH, et/ou
      - un radical di-yle aryle en particulier phényle, ou
   - une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier de type polyalkylène glycol, et notamment une chaîne de type polyéthylène glycol ou polysiloxane,
- m est un entier variant de 1 à 14 et de préférence égal à 1 ou 8, et
- B' et A' représentent respectivement un radical choisi parmi une fonction carboxylique et amine,
notamment à titre d'intermédiaire de synthèse d'un polymère conforme à l'invention et en particulier comprenant au moins une entité de formule (III) ou (IIIa).

De manière inattendue, les inventeurs ont, en outre, constaté que des polymères reproduisant la structure d'une entité oligomérique ou polymérique de formule (III) ou (IIIa) sont également accessibles via la copolymérisation d'au moins un monomère de formule (VI) : 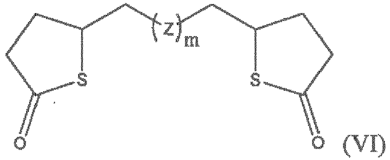 dans laquelle Z et m sont tels que définis en formule (III).

Les inventeurs ont également constaté que des polymères reproduisant la structure d'une entité oligomérique ou polymérique de formule (III) ou (IIIa) sont également accessibles via la copolymérisation d'au moins un monomère de formule (VII) : 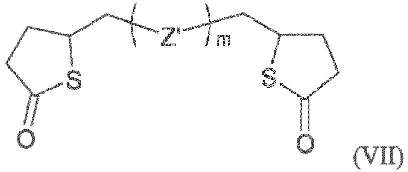 dans laquelle :
- Z' représente :
   - un motif hydrocarboné en C₁ à C₂₀, saturé ou insaturé, le cas échéant interrompu par :
      - un ou plusieurs hétéroatomes tels que des atomes d'oxygène, de soufre ou d'azote et de préférence un ou plusieurs atomes d'oxygène,
      - un ou plusieurs groupements divalents choisi parmi -NR'- avec R' étant un hydrogène ou un radical alkyle en C₁ à C₆, le cas échéant, substitué par une fonction -SH, et/ou
      - un radical di-yle aryle en particulier phényle,
   - un motif hydrocarboné en C₁ à C₂₀, saturé ou insaturé, le cas échéant interrompu par un ou plusieurs hétéroatomes tels que des atomes d'oxygène, de soufre ou d'azote et de préférence un ou plusieurs atomes d'oxygène, et substitué par un ou plusieurs groupements R₁, R₁ représentant un motif hydrocarboné en C₁ à C₂₀, saturé ou insaturé, le cas échéant interrompu par un ou plusieurs hétéroatomes tels que des atomes d'oxygène, de soufre ou d'azote et de préférence un ou plusieurs atomes d'oxygène, et pouvant être éventuellement substitué par un ou plusieurs groupes γ-thiolactone,
   - un ou plusieurs groupements divalents choisi parmi -NR"- avec R" étant un hydrogène, un groupe γ-thiolactone ou un radical alkyle en C₁ à C₆, le cas échéant, substitué par un groupe γ-thiolactone,
   - un radical -NR'-aryle-NR'-, et en particulier -NR'-phényle-NR'-, avec R' étant tel que défini ci-dessus,
   - un radical -O-aryle-O-, et en particulier -O-phényle-O-, le radical aryle, et en particulier phényle, étant éventuellement substitué par un ou plusieurs groupements R₁, R₁ étant tel que défini ci-dessus, ou
   - une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier du type polyalkylène glycol et, notamment, une chaîne du type polyéthylène glycol ou polysiloxane.
- m est un entier variant de 0 à 14, en particulier de 1 à 14, et de préférence égal à 1 ou 8.

De préférence, dans le monomère de formule (VII) :
- Z' représente :
   - un motif hydrocarboné en C₁ à C₂₀, saturé ou insaturé, le cas échéant interrompu par :
      - un ou plusieurs hétéroatomes tels que des atomes d'oxygène, de soufre ou d'azote et de préférence un ou plusieurs atomes d'oxygène,
      - un ou plusieurs groupements divalents choisi parmi NR'- avec R' étant un hydrogène ou un radical alkyle en C₁ à C₆, le cas échéant, substitué par une fonction -SH, et/ou
      - un radical di-yle aryle en particulier phényle, ou
   - une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier du type polyalkylène glycol et, notamment, une chaîne du type polyéthylène glycol ou polysiloxane,
- m est un entier variant de 1 à 14 et de préférence égal à 1 ou 8.

De préférence, un monomère de formule (VII) peut être choisi parmi les composés suivants :

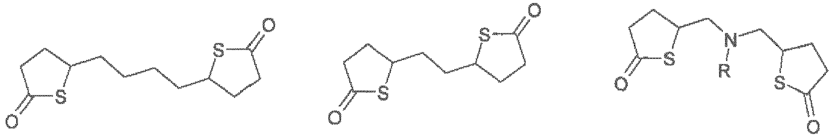

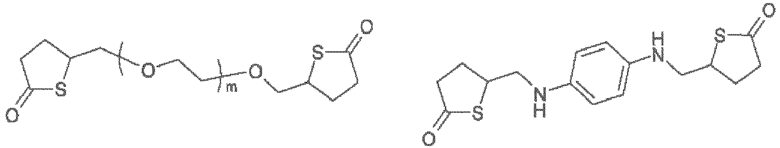

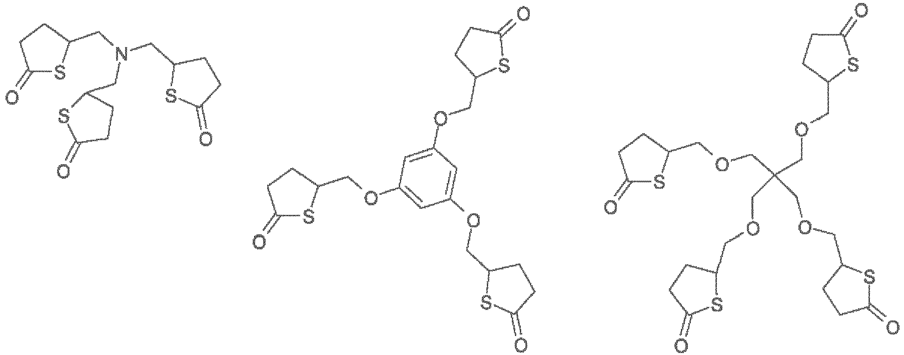

Les monomères de formules (VII) peuvent être notamment obtenus selon le procédé de préparation suivant : après une addition radicalaire d'un xanthate d'alkyl acétate sur une molécule portant des doubles liaisons en bout de chaînes en utilisant un peroxyde comme initiateur, le produit d'addition est saponifié en milieu basique pour obtenir une molécule ayant pour terminaison un ou plusieurs acides carboxyliques et une ou plusieurs fonctions SH en ω-4. Cet intermédiaire est ensuite mis en condition de cyclisation en présence d'acide en quantité catalytique pour donner une ou plusieurs fonctions thiolactone en bout de chaîne.

Avantageusement, les monomères de formules (VII) tels que définis ci-dessus peuvent être particulièrement utiles en tant que réactif avec des composés nucléophiles.

Ainsi selon un autre de ses aspects, l'invention concerne un procédé pour préparer un composé de formule (III) selon l'invention comprenant la condensation ou copolymérisation d'au moins une molécule de formules (VI) ou (VII) telles que définies ci-dessus, avec au moins un monomère choisi parmi H₂N-R-CO₂H, et H₂N-R-NH₂, dans lesquels R représente une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote.

Cette synthèse originale est notamment illustrée dans les exemples ci-après.

Comme précisé précédemment, les polymères selon l'invention sont particulièrement intéressants pour leurs propriétés en termes d'adhérence qui avantageusement s'avèrent modulables.

En effet, c'est la proportion en motifs de formule (I) au sein du polymère qui va contribuer à ses performances tackifiantes. Une faible proportion en motifs de formule (I) peut contribuer à former un revêtement amovible. A l'inverse, une proportion élevée contribue généralement à former un revêtement inamovible.

Ainsi un polymère selon l'invention peut posséder une proportion pondérale en motifs de formule (I) variant de 1 % à 90 % par rapport à son poids total.

Cette proportion en motifs de formule (I) peut ainsi être modulée lors de la copolymérisation des monomères dédiés à former l'entité structurelle de formule (II), (IIa), (III) et (IIIa) en ajustant la proportion des différents monomères mis en présence.

Toutefois, un polymère selon l'invention peut également dériver de la condensation ou copolymérisation d'au moins un précurseur d'une entité de formule (II), (IIa), (III) ou (IIIa) dans laquelle l'indice « n » a avantageusement une valeur différente de 1 et de préférence possède une valeur représentative d'un oligomère ou polymère avec au moins une chaine polymérique annexe et distincte.

Ainsi, selon un mode de réalisation, un polymère selon l'invention peut être formé d'au moins une entité de formule (II) ou (IIa) définies ci-dessus et/ou d'une entité de formule (III) ou (IIIa) définies ci-dessus sous forme condensée, notamment par copolymérisation, à au moins une chaine polymérique annexe distincte de ladite entité.

La chaîne polymérique annexe peut être choisie parmi les polyamides, les polyéthers, les chaînes siliconées, les polyamines et les polysulfures.

Cette condensation a pour effet de conférer à la chaîne polymérique considérée soit des propriétés tackifiantes si elle en est dénuée soit un gain en termes d'adhérence si elle possède déjà des propriétés tackifiantes.

Ainsi selon un de ses aspects, la présente invention a également pour objet un procédé utile pour moduler les propriétés d'adhérence d'un polymère comprenant la mise en présence dudit polymère avec au moins un précurseur d'une entité de formule (II), (IIa), (III) ou (IIIa) dans laquelle l'indice « n » a avantageusement une valeur différente de 1 et de préférence possède une valeur représentative d'un oligomère ou polymère, dans des conditions propices à la condensation et notamment à la copolymérisation dudit précurseur avec ledit polymère à traiter.

Comme précisé précédemment, la présente invention vise en outre une composition pour revêtement comprenant au moins un polymère conforme à l'invention.

Elle concerne également un support comprenant en surface au moins un revêtement dérivant d'un polymère selon l'invention.

Un tel revêtement peut notamment être obtenu par un procédé comprenant au moins la mise en contact de la surface à traiter dudit support avec au moins un polymère selon l'invention et l'exposition dudit polymère au contact dudit support à des conditions propices à sa transformation en un revêtement.

Ces conditions peuvent varier en fonction de la nature chimique du polymère voire de celle du support. Elles sont généralement efficaces pour provoquer la réticulation dudit polymère et l'établissement de liaisons, covalentes ou non, entre le revêtement ainsi formé et le support. Dans le cadre de la présente invention, les fonctions SH présentes dans le revêtement contribuent précisément efficacement à l'immobilisation de ce revêtement en surface du support. Ce phénomène est notamment vérifié dans les exemples présentés ci-après.

L'invention vise également un support traité en surface avec un revêtement dérivant en tout ou partie d'un polymère selon l'invention.

Les exemples figurant ci-après sont présentés à titre illustratif et non limitatif du domaine de l'invention.

### Exemple 1 :

### Synthèse de l'acide 12-amino-10-mercaptododécanoïque

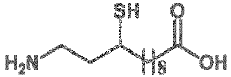

A une solution de 80 g d'acide 12-(1,3-dioxoisoindolin-2-yl)-10-((éthoxycarbonothioyl)thio) dodécanoïque (Réf. : S. Zard et al Organic Letters ; 2008, Vol. 10, p. 3 279) en solution dans 400 mL d'éthanol sont additionnés 40 mL d'hydrazine. Le mélange réactionnel est agité pendant 48 heures à température ambiante. Le solvant est ensuite évaporé. Le solide brut obtenu est lavé au diéthyl éther, puis recristallisé dans le méthanol.

### Exemple 2 :

### Synthèse du 5,5'-(butane-1,4-diyl)bis(dihydrothiophén-2(3H)-one)

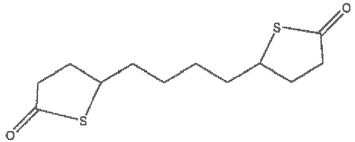

### Etape A : Synthèse du 4,9-bis((éthoxycarbonothioyl)thio)dodécanedioate de diéthyle

Un mélange de 15 g de 2-((éthoxycarbonothioyl)thio)acétate d'éthyl et de 5 mL de 1,7 octadiène est agité pendant 15 min à 125 °C sous atmosphère d'azote.

1,5 mL de péroxyde de di-terbutyl sont ensuite ajoutés et le mélange réactionnel est agité à 125 °C pendant 2 heures sous atmosphère d'azote. 1,5 ml de péroxyde de di-terbutyl sont alors à nouveau ajoutés au mélange réactionnel.

2 heures après la seconde addition de péroxyde, les produits secondaires sont évaporés et le brut réactionnel obtenu est utilisé sans autre purification pour l'étape suivante.

### Etape B : Synthèse de l'acide 4,9-dimercaptododécanedioïque

18 g du mélange brut obtenu à l'issue de l'étape A est mis en solution dans 100 mL d'éthanol. 100 mL d'eau sont ensuite ajoutés. La solution est ensuite dégazée en faisant buller de l'azote pendant 10 min. 15 g d'hydroxyde de sodium sont alors ajoutés et le mélange réactionnel agité pendant 2 heures à 60 °C.

L'éthanol est ensuite partiellement évaporé, la phase aqueuse basique est extraite avec du diéthyl éther puis acidifié jusqu'à pH=1 en ajoutant de l'acide chlorhydrique concentré. La phase aqueuse acide est alors extraite plusieurs fois avec du diéthyl éther.

Les phases organiques réunies sont séchées sur du sulfate de magnésium, puis filtrées et évaporées pour donner 11 g d'une huile brune qui sera utilisée sans autre purification pour l'étape suivante.

### Etape C : Synthèse de la 5,5'-(butane-1,4-diyil)bis(dihydrothiophén-2(3H)-one)

11 g du mélange brut obtenu à l'issue de l'étape B est mis en solution dans 100 mL de diéthyl éther. 10 gouttes d'acide sulfurique concentré sont ensuite ajoutées et le mélange agité au reflux du diethyl éther pendant 2 heures. 10 autres gouttes d'acide sulfurique concentré sont additionnées et le mélange agité au reflux du diethyl éther jusqu'à conversion totale.

Le diethyl éther est ensuite évaporé et le brut obtenu est chromatographié sur gel de silice avec un mélange 1 :1 d'éther de pétrole et de diéthyl éther comme éluants. Les 8 g de solide obtenus sont recristallisés dans de l'éthanol pour donner 5 g d'un solide blanc.

### Exemple 3 :

### Synthèse de la 5,5'-(éthane-1,2-diyl)bis(dihydrothiophén-2(3H)-one)

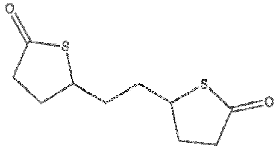

### Etape A : Synthèse du 4,7-bis((ethoxycarbonothioyl)thio)décanedioate de diéthyl diéthyl

Une solution de 10 g de 2-((éthoxycarbonothioyl)thio)acétate d'éthyl et 1,90 mL de 1,5- hexadiène dans 50 mL d'acétate d'éthyle est agitée pendant 10 min à reflux sous atmosphère d'azote.
1 g de péroxyde de dilauroyl est ensuite ajouté et le mélange réactionnel est agité au reflux pendant 1 heure et 30 minutes sous atmosphère d'azote. Deux fois 1 g de péroxyde de dilauroyl sont encore ajoutés toutes les 90 minutes.
2 heures après la dernière addition de péroxyde, le mélange réactionnel est évaporé à sec et le brut réactionnel obtenu est utilisé sans autre purification pour l'étape suivante.

### Etape B : Synthèse de l'acide 4,7-dimercaptodécanedioïque

14 g du mélange brut obtenu à l'issue de l'étape A sont mis en solution dans 100 mL d'éthanol. 100 mL d'eau sont ensuite ajoutés. La solution est ensuite dégazée en faisant buller de l'azote pendant 10 minutes. 9,6 g d'hydroxyde de sodium sont alors ajoutés et le mélange réactionnel agité pendant 2 heures à 60 °C.

L'éthanol est évaporé partiellement, la phase aqueuse basique est extraite avec du diéthyl éther puis acidifiée jusqu'à pH=1 en additionnant de l'acide chlorhydrique concentré. La phase aqueuse acide est alors extraite plusieurs fois avec du diéthyl éther.

Les phases organiques réunies sont séchées sur du sulfate de magnésium, puis filtrés et évaporées pour donner 7 g d'une huile brune qui sera utilisée sans autre purification pour l'étape suivante.

### Etape C : Synthèse de la 5,5'-(éthane-1,2-diyl)bis(dihydrothiophén-2(3H)-one)

7 g du mélange brut obtenu au stade B est mis en solution dans 100 mL de diéthyl éther. 20 gouttes d'acide sulfurique concentré sont ensuite ajoutées et le mélange agité au reflux du diéthyl éther pendant 4 heures.

Le diéthyl éther est ensuite évaporé et le brut obtenu est chromatographié sur gel de silice avec un mélange 1 :1 d'éther de pétrole et de diéthyl éther comme éluants. Les 4 g de solide obtenus sont recristallisés dans de l'éthanol pour donner 3 g d'un solide blanc.

### Exemple 4 :

### Synthèse du copolymère C₁₁-C₁₂(SH)

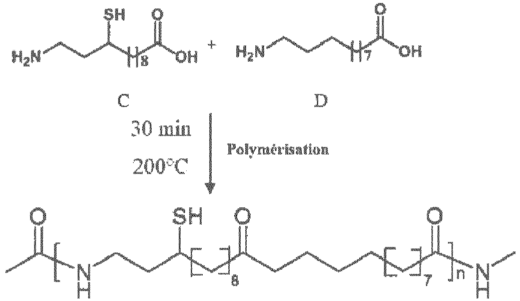

100 mg de C+D contenant différents pourcentages de B et C sont agités dans un tube en verre à une température de 200 °C pendant 30 minutes sous un flux d'azote. Le solide formé est ensuite démoulé à froid. La température de fusion des différents copolymères obtenus est mesurée.

| **% de C** | **T° Fusion** | **Polymérisation dans un tube en verre à 200 °C pendant 30 minutes** |
|---|---|---|
| **0 %** | 186 °C | Solide blanc qui se démoule du tube en verre |
| **1 %** | 193-195 °C | Solide blanc qui se démoule du tube en verre |
| **2 %** | 193-195 °C | Solide blanc qui se démoule du tube en verre |
| **5 %** | 195-200 °C | Solide blanc qui se démoule du tube en verre |
| **10 %** | ne fond pas à 300 °C | Solide qui commence à coller aux parois du tube |
| **20 %** | ne fond pas à 300 °C | Solide qui commence à coller aux parois du tube |
| **30 %** | ne fond pas à 300 °C | Solide qui colle complètement aux parois du tube |
| **100 %** | ne fond pas à 300 °C | Solide qui colle complètement aux parois du tube |

### Exemple 5 :

### Synthèse du copolymère polyamide PA6-12(2SH)

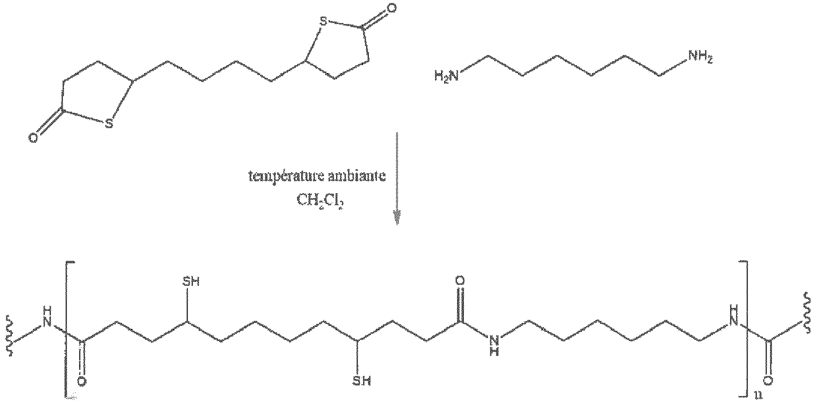

A 1 g de 5,5'-(butane-1,4-diyl)bis(dihydrothiophen-2(3H)-one) en solution dans 20 mL de dichlorométhane sont ajoutés 550 mg de 1,6-hexane diamine. Le mélange est agité à température ambiante pendant 24 heures. Le solvant est ensuite évaporé et 1,550 g d'un solide de couleur beige est obtenu.

Ce solide est posé sur une plaque de métal chauffée à 200 °C, puis une plaque de verre est appliquée dessus pendant 30 secondes.

Le dispositif est laissé refroidir à température ambiante pendant 24 heures. Le verre a bien adhéré au métal et ne peut être retiré par une force de traction manuelle.

### Exemple 6 :

### Synthèse du copolymère polyamide PA pipérazine-12(2S)

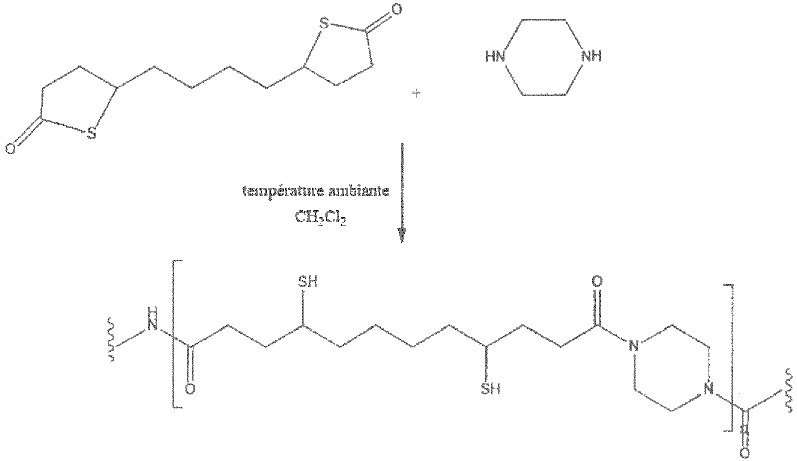

A 1 g de 5,5'-(butane-1,4-diyl)bis(dihydrothiophén-2(3H)-one) en solution dans 20 mL de dichlorométhane sont ajoutés 335 mg de pipérazine. Le mélange est agité au reflux pendant 1 heure. Le solvant est ensuite évaporé et 1,3 g d'une gomme collante de couleur miel est obtenue.

## Revendications

1. Polymère doté de propriétés d'adhérence comprenant au moins un, de préférence plusieurs, motifs structuraux, consécutifs ou non, de formule (I) : 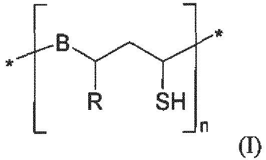 dans laquelle :
- B représente un motif carbonyle ou -NR'- avec R' représentant un atome d'hydrogène, un radical alkyle en C₁ à C₆, un radical acyle, carbamate ou aryle en C₅ à C₆ ; et
- R représente :
- un atome d'hydrogène,
- un radical hydrocarboné en C₁ à C₆, linéaire ou ramifié, saturé ou insaturé et, le cas échéant, substitué par un ou plusieurs atomes d'halogène,
- un radical aryle en C₅ à C₆, ou
- un radical acyle, acyloxy, alcoxycarbonyle ou cyano.

2. Polymère selon la revendication précédente, comprenant une quantité efficace en motifs de formule (I) pour lui conférer une propriété tackifiante.

3. Polymère selon l'une quelconque des revendications précédentes, dont la proportion pondérale en motifs de formule (I) varie de 1 % à 90% par rapport à son poids total.

4. Polymère selon l'une quelconque des revendications précédentes, dans lequel lesdits motifs de formule (I) n'y sont pas présents de manière contigüe.

5. Polymère selon l'une quelconque des revendications précédentes, dans lequel ledit motif de formule (I) est présent dans ledit polymère sous la forme d'une entité structurelle de nature oligomérique, polymérique ou non, avantageusement condensable notamment par copolymérisation.

6. Polymère selon l'une quelconque des revendications précédentes, comprenant au moins une entité de formule (II) : 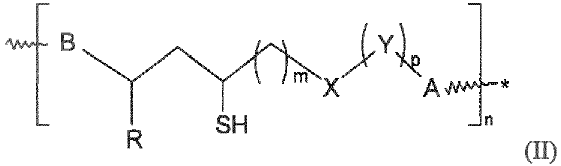 dans laquelle :
- m est un entier variant de 1 à 14 et de préférence égal à 1 ou 8,
- p est égale à zéro ou 1,
- n est un entier représentatif d'un enchaînement oligomérique ou polymérique, en particulier de PM pouvant varier de 2 à 2 000,
- R est tel que défini en revendication 1,
- X représente un motif carbonyle ou -NR'- avec R' représentant un atome d'hydrogène ou un radical alkyle en C₁ à C₆,
- Y représente un motif 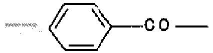 dont la fonction carbonyle est liée à A,
- A représente :
• un radical de formule -NR₁(R₂)CO- avec :
* R₂ figurant une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et
* R₁ représentant un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle, ou
• un radical de formule -NH(R")NH-, -CO(R")CO-, -O(R")O- ou -S(R")S-avec R" représentant une chaine hydrocarbonée, linéaire ou ramifié, saturée ou insaturée, polymérique ou non, le cas échéant interrompue par un ou plusieurs hétéroatomes, de préférence des atomes d'oxygène d'azote ou de soufre, un radical aryle ou aralkyle, et
- B représente un motif carbonyle ou -NR'- avec R' représentant un atome d'hydrogène ou un radical alkyle en C₁ à C₆ avec B étant apte à former un groupement de liaison avec la fonction terminale d'un motif A.

7. Polymère selon la revendication précédente, dans lequel l'entité de formule (II) répond à la formule (IIa) : 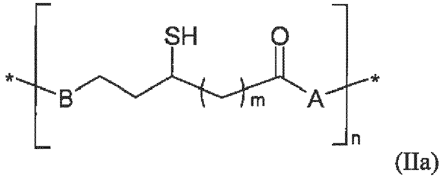 dans laquelle :
- n, m, et B sont tels que définis en revendication 6, et
- A représente :
• un radical de formule -NH(R₂)CO- avec R₂ figurant une chaîne hydrocarbonée de C₂ à C₁₀, linéaire ou ramifiée, le cas échéant, interrompue par un ou plusieurs hétéroatomes comme des atomes d'oxygène, de soufre ou d'azote,
• un radical de formule -NH(R")NH- avec R" représentant une chaine hydrocarbonée, de C₂ à C₁₀, linéaire ou ramifié, saturée ou insaturée, polymérique ou non, le cas échéant interrompue par un ou plusieurs hétéroatomes, de préférence des atomes d'oxygène, d'azote ou de soufre.

8. Polymère selon l'une quelconque des revendications précédentes, comprenant au moins une entité de formule (IIb) : 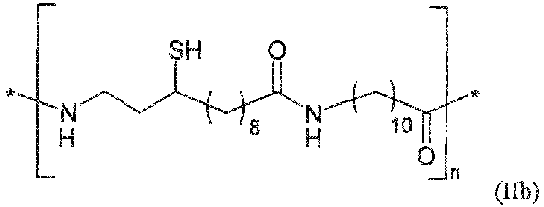 dans laquelle n est tel que défini en revendication 6.

9. Polymère selon l'une quelconque des revendications 1 à 5, comprenant au moins une entité de formule (III) : 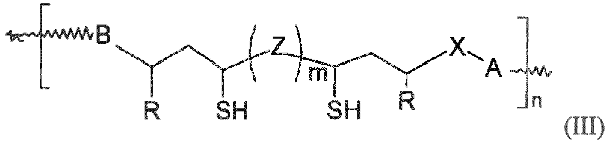 dans laquelle :
- n, m, B, X et R sont tels que définis en revendication 6,
- A représente :
• un radical de formule -NR₁(R')NR₂- avec R' figurant une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et R₁ et R₂ représentant, indépendamment l'un de l'autre, un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle,
• un radical di-yle hétérocyclique pouvant être choisi parmi les radicaux pipérazinyle, 1,3,4,5-tétrahydroimidazolyle, 1,4-diazépanyle, ou 1,5-diazocanyle,
• un radical de formule -CO(R")CO-, -O(R")O- ou -S(R")S- avec R" représentant une chaine hydrocarbonée, linéaire ou ramifiée, saturée ou insaturée, polymérique ou non, le cas échéant interrompue par un ou plusieurs hétéroatomes, de préférence des atomes d'oxygène, d'azote ou de soufre, un radical aryle ou aralkyle, et
- Z représente :
• un motif hydrocarboné en C₁ à C₂₀, saturé ou insaturé, le cas échéant interrompu par :
- un ou plusieurs hétéroatomes tels que des atomes d'oxygène, de soufre ou d'azote et de préférence un ou plusieurs atomes d'oxygène,
- un ou plusieurs groupements divalents choisi parmi -NR'- avec R' étant un hydrogène ou un radical alkyle en C₁ à C₆, le cas échéant, substitué par une fonction -SH, et/ou
- un radical di-yle aryle en particulier phényle, ou
• une chaine polymérique hydrocarbonée ou siliconée, le cas échéant interrompue par un on plusieurs hétéroatomes comme des atomes d'oxygène, de soufre ou d'azote et de préférence un ou plusieurs atomes d'oxygène, en particulier de type polyalkylène glycol.

10. Polymère selon la revendication précédente, dans lequel l'entité de formule (III) est de formule (IIIa) : 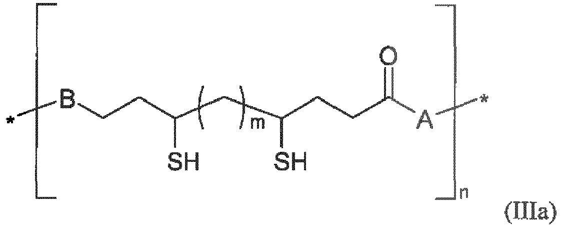 dans laquelle :
- n, m et B sont tels que définis en revendication 9, et
- A représente :
• un radical de formule -NR₁(R')NR₂- avec R' figurant une chaîne hydrocarbonée de C₂ à C₁₀, linéaire ou ramifiée, le cas échéant, interrompue par un ou plusieurs hétéroatomes comme des atomes d'oxygène, de soufre ou d'azote ou un radical cyclique, saturé, insaturé ou aromatique di-yle de préférence en para, et R₁ et R₂ représentant indépendamment l'un de l'autre : un atome d'hydrogène ou un radical alkyle en C₁ à C₆, acyclique ou cyclique, le cas échéant substitué, en particulier par un atome d'halogène, un radical acyle, un radical aryle, un radical aralkyle ou un hétérocycle, ou
• un radical di-yle hétérocyclique pouvant être choisi parmi les motifs pipérazinyle, 1,3,4,5-tétrahydroimidazolyle, 1,4-diazépanyle et 1,5-diazocanyle.

11. Polymère selon la revendication 9 ou 10, dans lequel l'entité (III) est de formule (IIIb) : 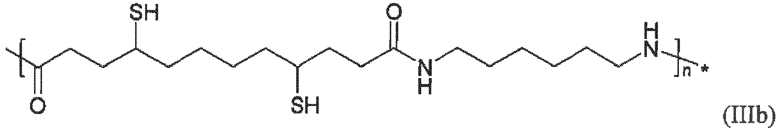 dans laquelle n est tel que défini en revendication 9.

12. Polymère selon la revendication 9 ou 10, dans lequel l'entité (III) est de formule (IIIc) : 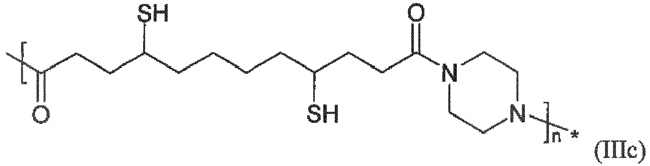 dans laquelle n est tel que défini en revendication 9.

13. Polymère selon l'une quelconque des revendications précédentes, comprenant au moins une entité de formule (II) ou (IIa) selon la revendication 6 à 8 ou une entité de formule (III) ou (IIIa) selon la revendication 9 à 12 sous forme condensée, notamment par copolymérisation, à au moins une chaine polymérique annexe distincte de ladite entité.

14. Polymère selon la revendication précédente, dans lequel la chaîne polymérique distincte est choisie parmi les polyamides, les polyéthers, les chaînes siliconées, les polyamines et les polysulfures.

15. Composition pour revêtement comprenant au moins un polymère selon l'une quelconque des revendications précédentes.

16. Procédé pour former un revêtement en surface d'un support comprenant au moins la mise en contact de la surface à traiter dudit support avec un polymère selon l'une quelconque des revendications 1 à 14 et l'exposition dudit polymère au contact dudit support à des conditions propices à sa transformation en un revêtement.

17. Support traité en surface avec un revêtement dérivant en tout ou partie d'un polymère selon l'une quelconque des revendications 1 à 14.

18. Procédé utile pour moduler les propriétés en termes d'adhérence d'un polymère comprenant la mise en présence dudit polymère avec au moins un précurseur d'une entité de formule (II), (IIa), (III) ou (IIIa) selon l'une des revendications 6 à 14 dans laquelle l'indice « n » a avantageusement une valeur différente de 1 et de préférence possède une valeur représentative d'un oligomère ou polymère, dans des conditions propices à la condensation dudit précurseur avec ledit polymère à traiter.

19. Procédé pour préparer un composé de formule (III) selon l'une quelconque des revendications 9 à 13, comprenant la condensation ou copolymérisation d'au moins une molécule de formule (VI) : 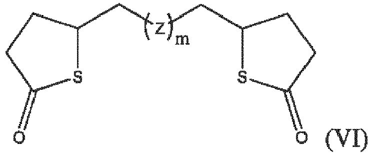 avec au moins un monomère choisi parmi H₂N-R-CO₂H, et H₂N-R-NH2 dans lesquels R représente une chaîne hydrocarbonée au moins en C₂, polymérique ou non, linéaire ou ramifiée, le cas échéant, interrompue par un ou plusieurs hétéroatomes comme des atomes d'oxygène, de soufre ou d'azote.

20. Composé de formule (V) : 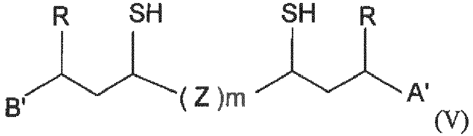 dans laquelle :
- R représente :
- un atome d'hydrogène,
- un radical hydrocarboné en C₁ à C₆, linéaire ou ramifié, saturé ou insaturé et, le cas échéant, substitué par un ou plusieurs atomes d'halogène,
- un radical aryle en C₅ à C₆, ou
- un radical acyle, acyloxy, alcoxycarbonyle ou cyano,
- Z représente :
• un motif hydrocarboné en C₁ à C₂₀, saturé ou insaturé, le cas échéant interrompu par :
- un ou plusieurs hétéroatomes comme des atomes d'oxygène, de soufre ou d'azote et de préférence un ou plusieurs atomes d'oxygène,
- un ou plusieurs groupements divalents choisi parmi -NR'- avec R' pouvant être un hydrogène ou un radical alkyle en C₁ à C₆, le cas échéant, substitué par une fonction -SH, et/ou
- un radical di-yle aryle, en particulier phényle, ou
• une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier de type polyalkylène glycol, et notamment une chaîne de type polyéthylène glycol ou polysiloxane,
- m est un entier variant de 1 à 14, et de préférence égal à 1 ou 8, et
- B' et A' représentent respectivement un radical choisi parmi une fonction carboxylique et amine.

21. Composé de formule (VII) : 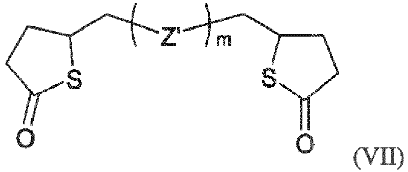 dans laquelle :
- Z' représente :
• un motif hydrocarboné en C₁ à C₂₀, saturé ou insaturé, le cas échéant interrompu par :
- un ou plusieurs hétéroatomes tels que des atomes d'oxygène, de soufre ou d'azote et de préférence un ou plusieurs atomes d'oxygène,
- un ou plusieurs groupements divalents choisi parmi NR'- avec R' étant un hydrogène ou un radical alkyle en C₁ à C₆, le cas échéant, substitué par une fonction -SH, et/ou
- un radical di-yle aryle en particulier phényle,
• un motif hydrocarboné en C₁ à C₂₀, saturé ou insaturé, le cas échéant interrompu par un ou plusieurs hétéroatomes tels que des atomes d'oxygène, de soufre ou d'azote et de préférence un ou plusieurs atomes d'oxygène, et substitué par un ou plusieurs groupements R₁, R₁ représentant un motif hydrocarboné en C₁ à C₂₀, saturé ou insaturé, le cas échéant interrompu par un ou plusieurs hétéroatomes tels que des atomes d'oxygène, de soufre ou d'azote et de préférence un ou plusieurs atomes d'oxygène, et pouvant être éventuellement substitué par un ou plusieurs groupes γ-thiolactone,
• un ou plusieurs groupements divalents choisi parmi -NR"- avec R" étant un hydrogène, un groupe γ-thiolactone ou un radical alkyle en C₁ à C₆, le cas échéant, substitué par un groupe γ-thiolactone,
• un radical -NR'-aryle-NR'-, et en particulier -NR'-phényle-NR'-, avec R' étant tel que défini ci-dessus,
• un radical -O-aryle-O-, et en particulier -O-phényle-O-, le radical aryle, et en particulier phényle, étant éventuellement substitué par un ou plusieurs groupements R₁, R₁ étant tel que défini ci-dessus, ou
• une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier du type polyalkylène glycol et, notamment, une chaîne du type polyéthylène glycol ou polysiloxane.
- m est un entier variant de 0 à 14, en particulier de 1 à 14, et de préférence égal à 1 ou 8.

22. Composé selon la revendication 21, dans laquelle :
- Z' représente :
• un motif hydrocarboné en C₁ à C₂₀, saturé ou insaturé, le cas échéant interrompu par :
- un ou plusieurs hétéroatomes tels que des atomes d'oxygène, de soufre ou d'azote et de préférence un ou plusieurs atomes d'oxygène,
- un ou plusieurs groupements divalents choisi parmi NR'- avec R' étant un hydrogène ou un radical alkyle en C₁ à C₆, le cas échéant, substitué par une fonction -SH, et/ou
- un radical di-yle aryle en particulier phényle, ou
• une chaine polymérique hydrocarbonée ou siliconée, le cas échéant interrompue par un ou plusieurs hétéroatomes comme des atomes d'oxygène, de soufre ou d'azote et de préférence un ou plusieurs atomes d'oxygène, en particulier du type polyalkylène glycol et, notamment, une chaîne du type polyéthylène glycol ou polysiloxane,
- m est un entier variant de 1 à 14 et de préférence égal à 1 ou 8.

23. Composé selon la revendication 21 ou 22, choisi parmi les composés suivants :

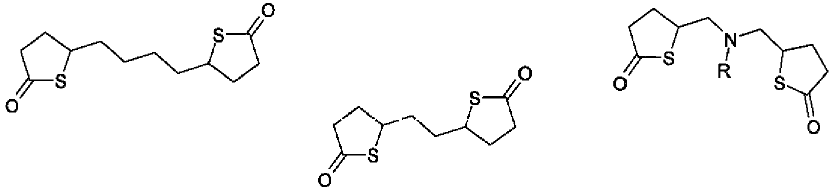

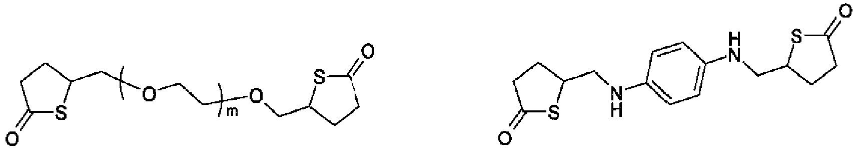

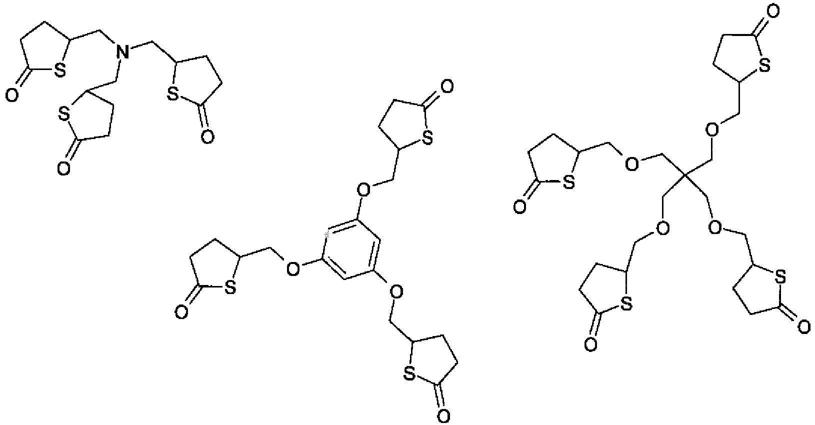

## Patentansprüche

1. Polymer mit Hafteigenschaften, umfassend mindestens eine und vorzugsweise mehrere aufeinanderfolgende oder nicht aufeinanderfolgende Strukturmotive der Formel (I): 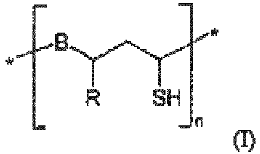 in der:
- B für ein Carbonyl- oder -NR'-Motiv steht, wobei R' für ein Wasserstoffatom, einen C₁- bis C₆-Alkylrest oder einen Acyl-, Carbamat- oder C₅- bis C₆-Arylrest steht; und
- R für:
- ein Wasserstoffatom,
- einen linearen oder verzweigten, gesättigten oder ungesättigten und gegebenenfalls durch ein oder mehrere Halogenatome substituierten C₁- bis C₆-Kohlenwasserstoffrest,
- einen C₅- bis C₆-Arylrest oder
- einen Acyl-, Acyloxy-, Alkoxycarbonyl- oder Cyanorest
steht.

2. Polymer nach dem vorhergehenden Anspruch, umfassend eine wirksame Menge an Motiven der Formel (I), um ihm eine klebrige Eigenschaft zu verleihen.

3. Polymer nach einem der vorhergehenden Ansprüche, dessen Gewichtsanteil an Motiven der Formel (I) von 1 % bis 90 %, bezogen auf sein Gesamtgewicht, variiert.

4. Polymer nach einem der vorhergehenden Ansprüche, wobei die Motive der Formel (I) nicht aneinander angrenzen.

5. Polymer nach einem der vorhergehenden Ansprüche, wobei das Motiv der Formel (I) in dem Polymer in Form einer gegebenenfalls oligomeren oder polymeren Struktureinheit, die vorteilhafterweise insbesondere durch Copolymerisation kondensierbar ist, vorliegt.

6. Polymer nach einem der vorhergehenden Ansprüche, umfassend mindestens eine Einheit der Formel (II): 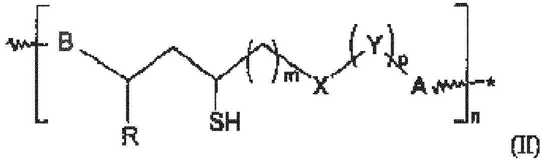 in der:
- m für eine ganze Zahl steht, die im Bereich von 1 bis 14 variiert und vorzugsweise gleich 1 bis 8 ist,
- p gleich 0 oder 1 ist,
- n für eine ganze Zahl steht, die für eine oligomere oder polymere Kette repräsentativ ist, insbesondere mit einem MW, das von 2 bis 2000 variieren kann,
- R wie in Anspruch 1 definiert ist,
- X für ein Carbonyl- oder -NR'-Motiv steht, wobei R' für ein Wasserstoffatom oder einen C₁- bis C₆-Alkylrest steht,
- Y für ein Motiv 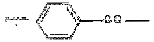 steht, dessen Carbonylfunktion an A gebunden ist,
- A für:
• einen Rest der Formel -NR₁(R₂)CO-, wobei:
* R₂ für eine lineare oder verzweigte, gegebenenfalls polymere Kohlenwasserstoffkette mit mindestens 2 C-Atomen, die gegebenenfalls durch ein oder mehrere Heteroatome wie Sauerstoff-, Schwefel- oder Stickstoffatome oder einen cyclischen, gesättigten, ungesättigten oder aromatischen Diylrest, vorzugsweise in para-Stellung, unterbrochen ist, steht und
* R₁ für ein Wasserstoffatom oder einen acyclischen oder cyclischen C₁- bis C₆-Alkylrest, der gegebenenfalls substituiert ist, insbesondere durch ein Halogenatom, einen Acylrest, einen Arylrest, einen Aralkylrest oder einen Heterocyclus, steht oder
• einen Rest der Formel -NH(R")NH-, -CO(R")CO-, -O(R")O- oder -S(R")S-, wobei R" für eine lineare oder verzweigte, gesättigte oder ungesättigte gegebenenfalls polymere Kohlenwasserstoffkette, die gegebenenfalls durch ein oder mehrere Heteroatome, vorzugsweise Sauerstoff-, Stickstoff- oder Schwefelatome, unterbrochen ist, oder einen Aryl- oder Aralkylrest steht,
steht und
- B für ein Carbonyl- oder -NR'-Motiv steht, wobei R' für ein Wasserstoffatom oder einen C₁- bis C₆-Alkylrest steht, wobei B mit der endständigen Funktion eines Motivs A eine Verbindungsgruppe bilden kann.

7. Polymer nach dem vorhergehenden Anspruch, wobei die Einheit der Formel (II) der Formel (IIa) entspricht: 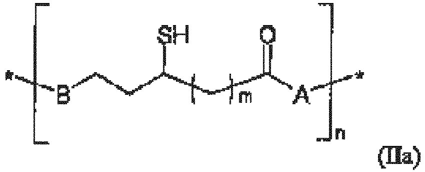 in der:
- n, m und B wie in Anspruch 6 definiert sind und
- A für:
• einen Rest der Formel -NH(R₂)CO-, wobei R₂ für eine lineare oder verzweigte C₂- bis C₁₀-Kohlenwasserstoffkette, die gegebenenfalls durch ein oder mehrere Heteroatome wie Sauerstoff-, Schwefel- oder Stickstoffatome unterbrochen ist, steht,
• einen Rest der Formel -NH(R")NH-, wobei R" für eine lineare oder verzweigte, gesättigte oder ungesättigte gegebenenfalls polymere C₂- bis C₁₀-Kohlenwasserstoffkette, die gegebenenfalls durch ein oder mehrere Heteroatome, vorzugsweise Sauerstoff-, Stickstoff- oder Schwefelatome, unterbrochen ist, steht,
steht.

8. Polymer nach einem der vorhergehenden Ansprüche, umfassend mindestens eine Einheit der Formel (IIb) : 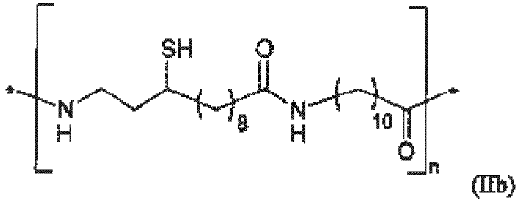 in der n wie in Anspruch 6 definiert ist.

9. Polymer nach einem der Ansprüche 1 bis 5, umfassend mindestens eine Einheit der Formel (III) : 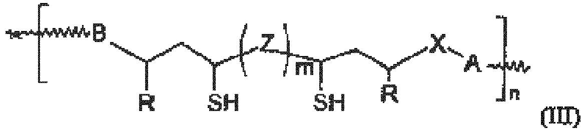 in der:
- n, m, B, X und R wie in Anspruch 6 definiert sind,
- A für:
• einen Rest der Formel -NR₁(R')NR₂-, wobei R' für eine lineare oder verzweigte, gegebenenfalls polymere Kohlenwasserstoffkette mit mindestens 2 C-Atomen, die gegebenenfalls durch ein oder mehrere Heteroatome wie Sauerstoff-, Schwefel- oder Stickstoffatome oder einen cyclischen, gesättigten, ungesättigten oder aromatischen Diylrest, vorzugsweise in para-Stellung, unterbrochen ist, steht und R₁ und R₂ unabhängig voneinander für ein Wasserstoffatom oder einen acyclischen oder cyclischen C₁- bis C₆-Alkylrest, der gegebenenfalls substituiert ist, insbesondere durch ein Halogenatom, einen Acylrest, einen Arylrest, einen Aralkylrest oder einen Heterocyclus, stehen oder
• einen heterocyclischen Diylrest, der aus Piperazinyl-, 1,3,4,5-Tetrahydroimidazolyl-, 1,4-Diazepanyl- oder 1,5-Diazocanylresten ausgewählt sein kann,
• einen Rest der Formel -CO(R")CO-, -O(R")O- oder -S(R")S-, wobei R" für eine lineare oder verzweigte, gesättigte oder ungesättigte gegebenenfalls polymere Kohlenwasserstoffkette, die gegebenenfalls durch ein oder mehrere Heteroatome, vorzugsweise Sauerstoff-, Stickstoff- oder Schwefelatome, unterbrochen ist, oder einen Aryl- oder Aralkylrest steht,
steht und
- Z für:
• ein gesättigtes oder ungesättigtes C₁- bis C₂₀-Kohlenwasserstoffmotiv, das gegebenenfalls durch
- ein oder mehrere Heteroatome wie Sauerstoff-, Schwefel- oder Stickstoffatome und vorzugsweise ein oder mehrere Sauerstoffatome,
- eine oder mehrere zweiwertige Gruppen, die aus -NR'- ausgewählt sind, wobei R' für Wasserstoff oder einen C₁- bis C₆-Alkylrest, der gegebenenfalls durch eine -SH-Funktion substituiert ist, steht, und/oder
- einen Aryl- und insbesondere Phenyldiylrest unterbrochen ist, oder
• eine Kohlenwasserstoff- oder Silikon-Polymerkette, die gegebenenfalls durch ein oder mehrere Heteroatome wie Sauerstoff-, Schwefel- oder Stickstoffatome und vorzugsweise ein oder mehrere Sauerstoffatome unterbrochen ist, insbesondere vom Polyalkylenglykol-Typ,
steht.

10. Polymer nach dem vorhergehenden Anspruch, wobei die Einheit der Formel (III) die Formel (IIIa) aufweist: 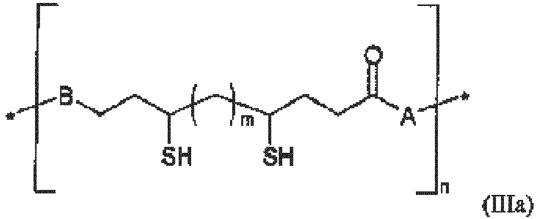 in der:
- n, m und B wie in Anspruch 9 definiert sind und
- A für:
• einen Rest der Formel -NR₁(R')NR₂-, wobei R' für eine lineare oder verzweigte Kohlenwasserstoffkette mit 2 bis 10 C-Atomen, die gegebenenfalls durch ein oder mehrere Heteroatome wie Sauerstoff-, Schwefel- oder Stickstoffatome oder einen cyclischen, gesättigten, ungesättigten oder aromatischen Diylrest, vorzugsweise in para-Stellung, unterbrochen ist, steht und R₁ und R₂ unabhängig voneinander für ein Wasserstoffatom oder einen acyclischen oder cyclischen C₁- bis C₆-Alkylrest, der gegebenenfalls substituiert ist, insbesondere durch ein Halogenatom, einen Acylrest, einen Arylrest, einen Aralkylrest oder einen Heterocyclus, stehen oder
• einen heterocyclischen Diylrest, der aus Piperazinyl-, 1,3,4,5-Tetrahydroimidazolyl-, 1,4-Diazepanyl- oder 1,5-Diazocanylresten ausgewählt sein kann,
steht.

11. Polymer nach Anspruch 9 oder 10, wobei die Einheit (III) die Formel (IIIb) aufweist: 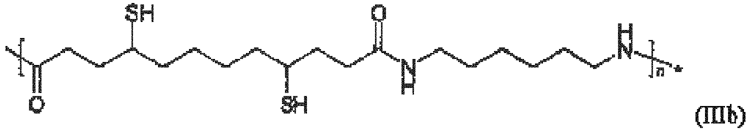 in der n wie in Anspruch 9 definiert ist.

12. Polymer nach Anspruch 9 oder 10, wobei die Einheit (III) die Formel (IIIc) aufweist: 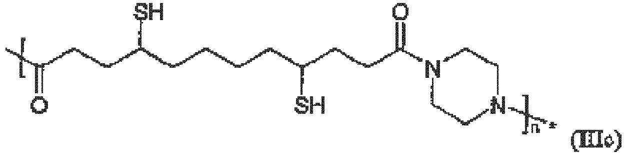 in der n wie in Anspruch 9 definiert ist.

13. Polymer nach einem der vorhergehenden Ansprüche, umfassend mindestens eine Einheit der Formel (II) oder (IIa) nach Anspruch 6 bis 8 oder eine Einheit der Formel (III) oder (IIIa) nach Anspruch 9 bis 12 in mit mindestens einer angehängten Polymerkette, die von der Einheit verschieden ist, kondensierter, insbesondere durch Copolymerisation kondensierter, Form.

14. Polymer nach dem vorhergehenden Anspruch, wobei die verschiedene Polymerkette aus Polyamiden, Polyethern, Silikonketten, Polyaminen und Polysulfiden ausgewählt ist.

15. Beschichtungszusammensetzung, umfassend mindestens ein Polymer nach einem der vorhergehenden Ansprüche.

16. Verfahren zur Bildung einer Beschichtung auf der Oberfläche eines Trägers, bei dem man mindestens die zu behandelnde Oberfläche des Trägers mit einem Polymer nach einem der Ansprüche 1 bis 14 in Kontakt bringt und das Polymer in Kontakt mit dem Träger Bedingungen aussetzt, die für seine Umwandlung in eine Beschichtung günstig sind.

17. Träger, der mit einer Beschichtung, die sich ganz oder teilweise von einem Polymer nach einem der Ansprüche 1 bis 14 ableitet, oberflächenbehandelt ist.

18. Verfahren zur Modulierung der Haftungseigenschaften eines Polymers, bei dem man das Polymer mit mindestens einem Vorläufer einer Einheit der Formel (II), (IIa), (III) oder (IIIa) nach einem der Ansprüche 6 bis 14, wobei der Index "n" vorteilhafterweise einen von 1 verschiedenen Wert hat und vorzugsweise einen Wert, der für ein Oligomer oder Polymer repräsentativ ist, besitzt, unter Bedingungen, die für die Kondensation des Vorläufers mit dem zu behandelnden Polymer günstig sind, in Berührung bringt.

19. Verfahren zur Herstellung einer Verbindung der Formel (III) nach einem der Ansprüche 9 bis 13, bei dem man mindestens ein Molekül der Formel (VI) : 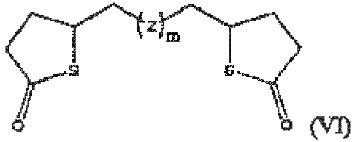 mit mindestens einem Monomer, das aus H₂N-R-CO₂H and H₂N-R-NH₂ ausgewählt ist, wobei R für eine lineare oder verzweigte, gegebenenfalls polymere Kohlenwasserstoffkette mit mindestens 2 C-Atomen, die gegebenenfalls durch ein oder mehrere Heteroatome wie Sauerstoff-, Schwefel- oder Stickstoffatome unterbrochen ist, steht, kondensiert oder copolymerisiert.

20. Verbindung der Formel (V): 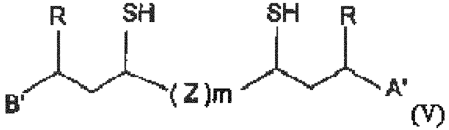 in der:
- R für:
- ein Wasserstoffatom,
- einen linearen oder verzweigten, gesättigten oder ungesättigten und gegebenenfalls durch ein oder mehrere Halogenatome substituierten C₁- bis C₆-Kohlenwasserstoffrest,
- einen C₅- bis C₆-Arylrest oder
- einen Acyl-, Acyloxy-, Alkoxycarbonyl- oder Cyanorest
steht,
- Z für:
• ein gesättigtes oder ungesättigtes C₁- bis C₂₀-Kohlenwasserstoffmotiv, das gegebenenfalls durch
- ein oder mehrere Heteroatome wie Sauerstoff-, Schwefel- oder Stickstoffatome und vorzugsweise ein oder mehrere Sauerstoffatome,
- eine oder mehrere zweiwertige Gruppen, die aus -NR'- ausgewählt sind, wobei R' für Wasserstoff oder einen C₁- bis C₆-Alkylrest, der gegebenenfalls durch eine -SH-Funktion substituiert ist, steht, und/oder
- einen Aryl- und insbesondere Phenyldiylrest unterbrochen ist, oder
• eine Kohlenwasserstoff- oder Silikon-Polymerkette, die gegebenenfalls durch ein oder mehrere Heteroatome wie Sauerstoff-, Schwefel- oder Stickstoffatome und vorzugsweise ein oder mehrere Sauerstoffatome unterbrochen ist, insbesondere vom Polyalkylenglykol-Typ, und im Besonderen eine Kette vom Polyethylenglykol- oder Polysiloxan-Typ
steht,
- m für eine ganze Zahl steht, die im Bereich von 1 bis 14 variiert und vorzugsweise gleich 1 bis 8 ist, und
B' und A' jeweils für einen Rest, der aus einer Carboxyl- und Aminfunktion ausgewählt ist, stehen.

21. Verbindung der Formel (VII): 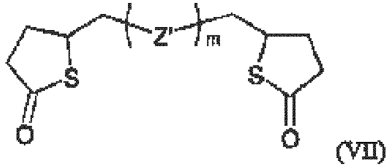 in der:
- Z' für:
• ein gesättigtes oder ungesättigtes C₁- bis C₂₀-Kohlenwasserstoffmotiv, das gegebenenfalls durch
- ein oder mehrere Heteroatome wie Sauerstoff-, Schwefel- oder Stickstoffatome und vorzugsweise ein oder mehrere Sauerstoffatome,
- eine oder mehrere zweiwertige Gruppen, die aus -NR'- ausgewählt sind, wobei R' für Wasserstoff oder einen C₁- bis C₆-Alkylrest, der gegebenenfalls durch eine -SH-Funktion substituiert ist, steht, und/oder
- einen Aryl- und insbesondere Phenyldiylrest unterbrochen ist,
• ein gesättigtes oder ungesättigtes C₁- bis C₂₀-Kohlenwasserstoffmotiv, das gegebenenfalls durch ein oder mehrere Heteroatome wie Sauerstoff-, Schwefel- oder Stickstoffatome und vorzugsweise ein oder mehrere Sauerstoffatome unterbrochen und durch eine oder mehrere Gruppen R₁ substituiert ist, wobei R₁ für ein gesättigtes oder ungesättigtes C₁- bis C₂₀-Kohlenwasserstoffmotiv, das gegebenenfalls durch ein oder mehrere Heteroatome wie Sauerstoff-, Schwefel- oder Stickstoffatome und vorzugsweise ein oder mehrere Sauerstoffatome unterbrochen ist und gegebenenfalls durch eine oder mehrere γ-Thiolactongruppen substituiert sein kann, steht,
• eine oder mehrere zweiwertige Gruppen, die aus -NR"- ausgewählt sind, wobei R" für Wasserstoff, eine γ-Thiolactongruppe oder einen C₁-bis C₆-Alkylrest, der gegebenenfalls durch eine γ-Thiolactongruppe substituiert ist, steht,
• einen -NR'-Aryl-NR'- und insbesondere -NR'-Phenyl-NR'-Rest, wobei R' wie oben definiert ist,
• einen -O-Aryl-O- und insbesondere -O-Phenyl-O-Rest, wobei der Aryl- und insbesondere Phenylrest gegebenenfalls durch eine oder mehrere Gruppen R₁ substituiert ist, wobei R₁ wie oben definiert ist, oder
• eine Kohlenwasserstoff- oder Silikon-Polymerkette, die gegebenenfalls durch ein oder mehrere Heteroatome wie Sauerstoff-, Schwefel- oder Stickstoffatome und vorzugsweise ein oder mehrere Sauerstoffatome unterbrochen ist, insbesondere vom Polyalkylenglykol-Typ, und im Besonderen eine Kette vom Polyethylenglykol- oder Polysiloxan-Typ
steht,
- m für eine ganze Zahl steht, die im Bereich von 0 bis 14, insbesondere von 1 bis 14, variiert und vorzugsweise gleich 1 bis 8 ist.

22. Verbindung nach Anspruch 21, in der:
- Z' für:
• ein gesättigtes oder ungesättigtes C₁- bis C₂₀-Kohlenwasserstoffmotiv, das gegebenenfalls durch
- ein oder mehrere Heteroatome wie Sauerstoff-, Schwefel- oder Stickstoffatome und vorzugsweise ein oder mehrere Sauerstoffatome,
- eine oder mehrere zweiwertige Gruppen, die aus -NR'- ausgewählt sind, wobei R' für Wasserstoff oder einen C₁- bis C₆-Alkylrest, der gegebenenfalls durch eine -SH-Funktion substituiert ist, steht, und/oder
- einen Aryl- und insbesondere Phenyldiylrest unterbrochen ist, oder
• eine Kohlenwasserstoff- oder Silikon-Polymerkette, die gegebenenfalls durch ein oder mehrere Heteroatome wie Sauerstoff-, Schwefel- oder Stickstoffatome und vorzugsweise ein oder mehrere Sauerstoffatome unterbrochen ist, insbesondere vom Polyalkylenglykol-Typ, und im Besonderen eine Kette vom Polyethylenglykol- oder Polysiloxan-Typ
steht,
- m für eine ganze Zahl steht, die im Bereich von 1 bis 14 variiert und vorzugsweise gleich 1 bis 8 ist.

23. Verbindung nach Anspruch 21 oder 22, die aus den folgenden Verbindungen ausgewählt ist:

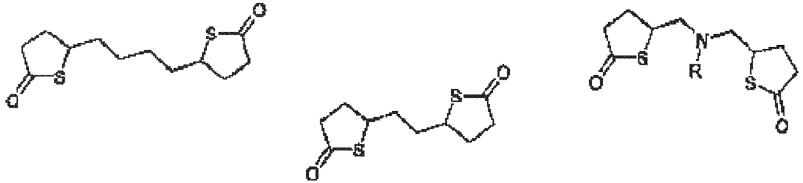

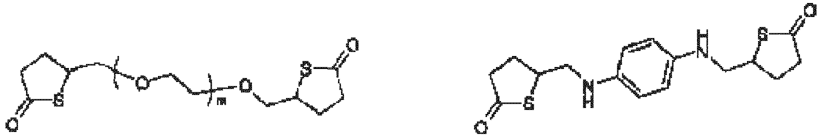

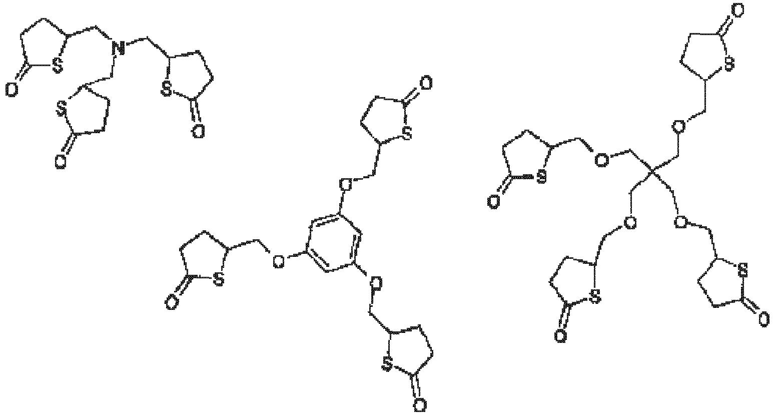

## Claims

1. Polymer having adhesion properties, comprising at least one, preferably several, consecutive or nonconsecutive structural units of formula (I): 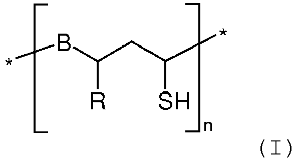 in which:
- B represents a carbonyl or -NR'- unit with R' representing a hydrogen atom, a C₁ to C₆ alkyl radical, or a C₅ to C₆ aryl, carbamate or acyl radical; and
- R represents:
- a hydrogen atom,
- a linear or branched, saturated or unsaturated C₁ to C₆ hydrocarbon-based radical which, where appropriate, is substituted with one or more halogen atoms,
- a C₅ to C₆ aryl radical, or
- an acyl, acyloxy, alkoxycarbonyl or cyano radical.

2. Polymer according to the preceding claim, comprising an effective amount of units of formula (I) to confer thereon a tackifying property.

3. Polymer according to either one of the preceding claims, of which the weight proportion of units of formula (I) ranges from 1% to 90% relative to its total weight.

4. Polymer according to any one of the preceding claims, in which said units of formula (I) are not present therein in a contiguous manner.

5. Polymer according to any one of the preceding claims, wherein said unit of formula (I) is present in said polymer in the form of a structural entity, of optionally polymeric or oligomeric nature, advantageously condensable in particular by copolymerization.

6. Polymer according to any one of the preceding claims, comprising at least one entity of formula (II): 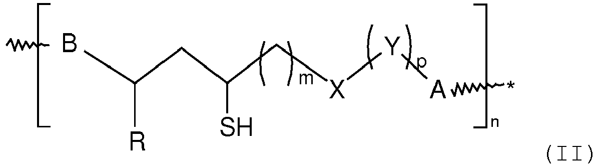 in which:
- m is an integer ranging from 1 to 14 and preferably equal to 1 or 8,
- p is equal to zero or 1,
- n is an integer representative of an oligomeric or polymeric sequence, in particular having a MW which may range from 2 to 2000,
- R is as defined in Claim 1,
- X represents a carbonyl or -NR'- unit with R' representing a hydrogen atom or a C₁ to C₆ alkyl radical,
- Y represents a unit 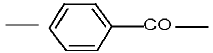 of which the carbonyl function is bonded to A,
- A represents:
• a radical of formula -NR₁(R₂)CO- with:
* R₂ representing a linear or branched, polymeric or non-polymeric, at least C₂ hydrocarbon-based chain, where appropriate interrupted with one or more heteroatoms such as oxygen, sulfur or nitrogen atoms or a cyclic, saturated or unsaturated or aromatic diyl radical preferably in the para-position, and
* R₁ representing a hydrogen atom or an acyclic or cyclic C₁ to C₆ alkyl radical, where appropriate substituted, in particular with a halogen atom, an acyl radical, an aryl radical, an aralkyl radical or a heterocycle, or
• a radical of formula -NH(R")NH-, -CO(R")CO-,-O(R")O- or -S(R")S-, with R" representing a linear or branched, saturated or unsaturated, polymeric or non-polymeric hydrocarbon-based chain, where appropriate interrupted with one or more heteroatoms, preferably oxygen, nitrogen or sulfur atoms, or an aryl or aralkyl radical, and
- B represents a carbonyl or -NR'- unit with R' representing a hydrogen atom or a C₁ to C₆ alkyl radical with B being capable of forming a bonding group with the end function of a unit A.

7. Polymer according to the preceding claim, wherein the entity of formula (II) corresponds to formula (IIa) : 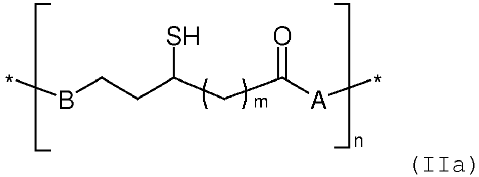 in which:
- n, m and B are as defined in Claim 6, and
- A represents:
• a radical of formula -NH(R₂)CO- with R₂ representing a linear or branched C₂ to C₁₀ hydrocarbon-based chain, where appropriate interrupted with one or more heteroatoms such as oxygen, sulfur or nitrogen atoms,
• a radical of formula -NH(R")NH- with R" representing a linear or branched, saturated or unsaturated, polymeric or non-polymeric, C₂ to C₁₀ hydrocarbon-based chain, where appropriate interrupted with one or more heteroatoms, preferably oxygen, nitrogen or sulfur atoms.

8. Polymer according to any one of the preceding claims, comprising at least one entity of formula (IIb) : 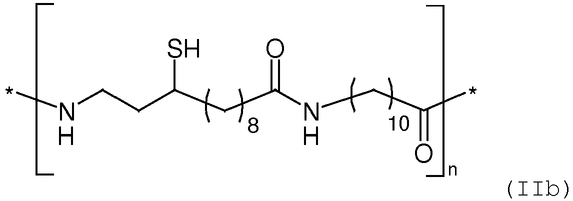 in which n is as defined in Claim 6.

9. Polymer according to any one of Claims 1 to 5, comprising at least one entity of formula (III): 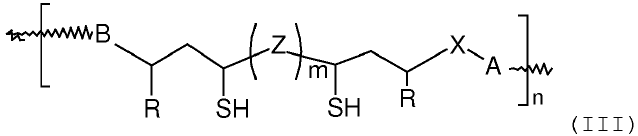 in which:
- n, m, B, X and R are as defined in Claim 6,
- A represents:
• a radical of formula -NR₁(R')NR₂- with R' representing a linear or branched, polymeric or non-polymeric, at least C₂ hydrocarbon-based chain, where appropriate interrupted with one or more heteroatoms such as oxygen, sulfur or nitrogen atoms or a cyclic, saturated, unsaturated or aromatic diyl radical, preferably in the para-position, and R₁ and R₂ represent, independently of one another, a hydrogen atom or an acyclic or cyclic C₁ to C₆ alkyl radical, where appropriate substituted, in particular with a halogen atom, an acyl radical, an aryl radical, an aralkyl radical or a heterocycle,
• a heterocyclic diyl radical which may be chosen from piperazinyl, 1,3,4,5-tetrahydroimidazolyl, 1,4-diazepanyl or 1,5-diazocanyl radicals,
• a radical of formula -CO(R")CO-, -O(R")O- or-S(R")S- with R" representing a linear or branched, saturated or unsaturated, polymeric or non-polymeric hydrocarbon-based chain, where appropriate interrupted with one or more heteroatoms, preferably oxygen, nitrogen or sulfur atoms, or an aryl or aralkyl radical, and
- Z represents:
• a saturated or unsaturated C₁ to C₂₀ hydrocarbon-based unit, where appropriate interrupted with:
- one or more heteroatoms such as oxygen, sulfur or nitrogen atoms, and preferably one or more oxygen atoms,
- one or more divalent groups chosen from -NR'-with R' being a hydrogen atom or a C₁ to C₆ alkyl radical, where appropriate substituted with an -SH function, and/or
- an aryl diyl radical, in particular phenyl,
or
• a hydrocarbon-based or silicone-based polymeric chain, where appropriate interrupted with one or more heteroatoms such as oxygen, sulfur or nitrogen atoms and preferably one or more oxygen atoms, in particular of the polyalkylene glycol type.

10. Polymer according to the preceding claim, wherein the entity of formula (III) is of formula (IIIa) : 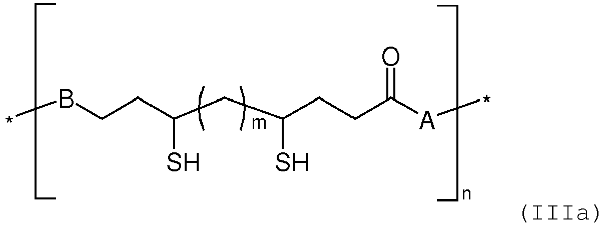 in which:
- n, m and B are as defined in Claim 9, and
- A represents:
• a radical of formula -NR₁(R')NR₂- with R' representing a linear or branched C₂ to C₁₀ hydrocarbon-based chain, where appropriate interrupted with one or more heteroatoms such as oxygen, sulfur or nitrogen atoms or a cyclic, saturated, unsaturated or aromatic diyl radical, preferably in the para-position, and R₁ and R₂ representing, independently of one another, a hydrogen atom or an acyclic or cyclic C₁ to C₆ alkyl radical, where appropriate substituted, in particular with a halogen atom, an acyl radical, an aryl radical, an aralkyl radical or a heterocycle, or
• a heterocyclic diyl radical which may be chosen from piperazinyl, 1,3,4,5-tetrahydroimidazolyl, 1,4-diazepanyl and 1,5-diazocanyl units.

11. Polymer according to Claim 9 or 10, wherein the entity (III) is of formula (IIIb): 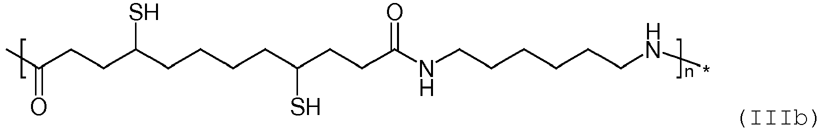 in which n is as defined in Claim 9.

12. Polymer according to Claim 9 or 10, wherein the entity (III) is of formula (IIIc): 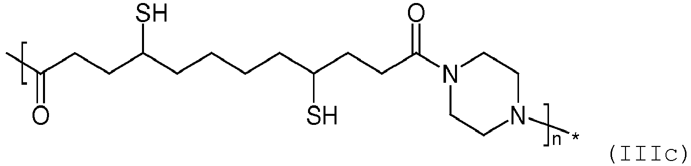 in which n is as defined in Claim 9.

13. Polymer according to any one of the preceding claims, comprising at least one entity of formula (II) or (IIa) according to Claims 6 to 8 or an entity of formula (III) or (IIIa) according to Claims 9 to 12 in a form condensed, in particular by copolymerization, to at least one distinct secondary polymeric chain of said entity.

14. Polymer according to the preceding claim, wherein the distinct polymeric chain is chosen from polyamides, polyethers, silicone-based chains, polyamines and polysulfides.

15. Composition for coating, comprising at least one polymer according to any one of the preceding claims.

16. Process for forming a coating at the surface of a support, comprising at least bringing the surface to be treated of said support into contact with a polymer according to any one of Claims 1 to 14 and exposing said polymer in contact with said support to conditions suitable for its conversion into a coating.

17. Support surface-treated with a coating deriving totally or partly from a polymer according to any one of Claims 1 to 14.

18. Process that is of use for modulating the properties in terms of adhesion of a polymer, comprising bringing said polymer into contact with at least one precursor of an entity of formula (II), (IIa), (III) or (IIIa) according to one of Claims 6 to 14 in which the index "n" advantageously has a value other than 1 and preferably has a value representative of an oligomer or polymer, under conditions suitable for the condensation of said precursor with said polymer to be treated.

19. Process for preparing a compound of formula (III) according to any one of Claims 9 to 13, comprising the condensation or copolymerization of at least one molecule of formula (VI): 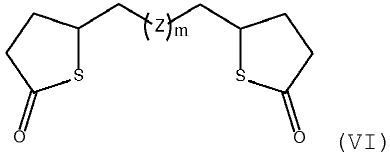 with at least one monomer chosen from H₂N-R-CO₂H and H₂N-R-NH₂ in which R represents a linear or branched, polymeric or non-polymeric, at least C₂ hydrocarbon-based chain, where appropriate interrupted with one or more heteroatoms such as oxygen, sulfur or nitrogen atoms.

20. Compound of formula (V): 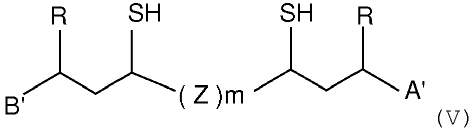 in which:
- R represents:
- a hydrogen atom,
- a linear or branched, saturated or unsaturated C₁ to C₆ hydrocarbon-based radical, where appropriate substituted with one or more halogen atoms,
- a C₅ to C₆ aryl radical, or
- an acyl, acyloxy, alkoxycarbonyl or cyano radical,
- Z represents:
• a saturated or unsaturated C₁ to C₂₀ hydrocarbon-based unit, where appropriate interrupted with:
- one or more heteroatoms such as oxygen, sulfur or nitrogen atoms and preferably one or more oxygen atoms,
- one or more divalent groups chosen from -NR'-with R' possibly being a hydrogen atom or a C₁ to C₆ alkyl radical, where appropriate substituted with an-SH function, and/or
- an aryl diyl radical, in particular phenyl,
or
• a hydrocarbon-based or silicone-based polymeric chain, where appropriate interrupted with one or more heteroatoms such as oxygen, sulfur or nitrogen atoms and preferably one or more oxygen atoms, in particular of polyalkylene glycol type, and in particular a chain of polyethylene glycol or polysiloxane type,
- m is an integer ranging from 1 to 14 and preferably equal to 1 or 8, and
- B' and A' represent respectively a radical chosen from a carboxylic and amine function.

21. Compound of formula (VII): 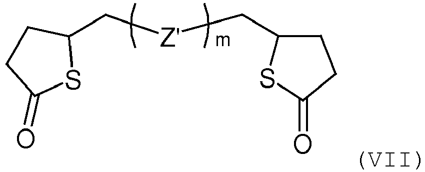 in which:
- Z' represents:
• a saturated or unsaturated C₁ to C₂₀ hydrocarbon-based unit, where appropriate interrupted with:
- one or more heteroatoms such as oxygen, sulfur or nitrogen atoms and preferably one or more oxygen atoms,
- one or more divalent groups chosen from -NR'-with R' being a hydrogen atom or a C₁ to C₆ alkyl radical, where appropriate substituted with an -SH function, and/or
- an aryl diyl radical, in particular phenyl,
• a saturated or unsaturated C₁ to C₂₀ hydrocarbon-based unit, where appropriate interrupted with one or more heteroatoms such as oxygen, sulfur or nitrogen atoms and preferably one or more oxygen atoms, and substituted with one or more R₁ groups, R₁ representing a saturated or unsaturated C₁ to C₂₀ hydrocarbon-based unit, where appropriate interrupted with one or more heteroatoms such as oxygen, sulfur or nitrogen atoms and preferably one or more oxygen atoms, and possibly being optionally substituted with one or more γ-thiolactone groups,
• one or more divalent groups chosen from -NR"- with R" being a hydrogen atom, a γ-thiolactone group or a C₁ to C₆ alkyl radical, where appropriate substituted with a γ-thiolactone group,
• an -NR'-aryl-NR'- radical, and in particular - NR'-phenyl-NR'- radical, with R' being as defined above,
• an -O-aryl-O- radical, and in particular -0-phenyl-O- radical, the aryl radical, and in particular phenyl radical, being optionally substituted with one or more R₁ groups, R₁ being as defined above, or
• a hydrocarbon-based or silicone-based polymeric chain, where appropriate interrupted with one or more heteroatoms such as oxygen, sulfur or nitrogen atoms and preferably one or more oxygen atoms, in particular of the polyalkylene glycol type and, in particular, a chain of the polyethylene glycol or polysiloxane type,
- m is an integer ranging from 0 to 14, in particular from 1 to 14, and preferably equal to 1 or 8.

22. Compound according to Claim 21, wherein:
- Z' represents:
• a saturated or unsaturated C₁ to C₂₀ hydrocarbon-based unit, where appropriate interrupted with:
- one or more heteroatoms such as oxygen, sulfur or nitrogen atoms and preferably one or more oxygen atoms,
- one or more divalent groups chosen from -NR'-with R' being a hydrogen atom or a C₁ to C₆ alkyl radical, where appropriate substituted with an -SH function, and/or
- an aryl diyl radical, in particular phenyl,
or
• a hydrocarbon-based or silicone-based polymeric chain, where appropriate interrupted with one or more heteroatoms such as oxygen, sulfur or nitrogen atoms and preferably one or more oxygen atoms, in particular of the polyalkylene glycol type and, in particular, a chain of the polyethylene glycol or polysiloxane type,
- m is an integer ranging from 1 to 14 and preferably equal to 1 or 8.

23. Compound according to Claim 21 or 22, chosen from the following compounds:
